(19) Europäisches Patentamt
European Patent Office
Office européen des brevets

(11) Publication number: **0 004 134**
**B1**

(12) **EUROPEAN PATENT SPECIFICATION**

(45) Date of publication of patent specification: **30.12.81**

(21) Application number: **79300230.4**

(22) Date of filing: **15.02.79**

(51) Int. Cl.³: **C 07 D 487/14,**
A 61 K 31/505,
//C 07 D 205/08

(54) **Beta-lactam antibacterial agents, their pharmaceutical compositions and process for their preparation.**

(30) Priority: **04.03.78 GB 866978**

(43) Date of publication of application:
**19.09.79 Bulletin 79/19**

(45) Publication of the grant of the European patent:
**30.12.81 Bulletin 81/52**

(84) Designated Contracting States:
**BE DE FR GB NL**

(56) References cited:
**FR - A - 2 246 547**
**GB - A - 1 455 016**

(73) Proprietor: **BEECHAM GROUP LIMITED**
**Beecham House Great West Road**
**Brentford, Middlesex (GB)**

(72) Inventor: **Pearson, Michael John**
**33 Greenfields Road Roffey**
**Horsham Surrey (GB)**

(74) Representative: **Cole, William Gwyn, et al,**
**Beecham Pharmaceuticals**
**Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 004 134 B1

β-lactam antibacterial agents, their pharmaceutical compositions and process for their preparation.

β-Lactam antibiotics such as penicillins and cephalosporins are bicyclic structures which contain a β-lactam ring fused to a further ring. A group of tricyclic β-lactam antibiotics has now been discovered.

Accordingly the present invention provides the compounds of the formula (I):

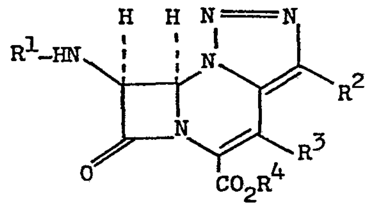

wherein $R^1$ is a hydrogen atom, a trityl group or an acyl group as found in known antibacterially active penicillins or cephalosporins; $R^2$ is a hydrogen atom, a lower alkyl group or an aryl group; $R^3$ is a hydrogen atom, a lower alkyl group, a substituted lower alkyl or a thiosubstituted lower alkyl group; and $R^4$ is a group such that $CO_2R^4$ is a carboxylic acid group or a salt or readily removable ester thereof.

When used herein the term "lower" means that the group contains up to 4 carbon atoms. When used herein the term "substituted" means substituted by a $CO_2R^4$ or $OR^5$ group where $R^4$ is as defined above and $R^5$ is a lower alkyl or lower acyl group. When used herein the term "aryl" means phenyl, pyridyl or phenyl substituted by a fluorine, chlorine or bromine atom or a nitro, lower alkyl, lower alkoxyl, carboxylic acid group or a lower alkyl or benzyl ester thereof. When used herein the term "thiosubstituted" means substituted by a group $SR^7$, where $R^7$ is a 5-membered heteroaromatic ring containing up to 4 heteroatoms any of which may be N, and one of which may be O or S and which may be optionally substituted by a lower alkyl group or a substituted lower alkyl group.

Those compounds of the formula (I) wherein $R^1$ is a hydrogen atom are intended mainly as intermediates in the preparation of corresponding compounds of the formula (I) wherein $R^1$ is an acyl group.

Those compounds of the formula (I) wherein $R^1$ is a trityl group are intended mainly as intermediates in the preparation of corresponding compounds of the formula (I) wherein $R^1$ is a hydrogen atom.

Those compounds of the formula (I) wherein $CO_2R^4$ is an esterified carboxyl group are primarily intended as intermediates in the preparation of a corresponding compound of the formula (I) wherein $CO_2R^4$ is a carboxylic acid group or a salt thereof.

From the foregoing it will be realized that the antibacterially active compounds of this invention can be represented by the formula (II):

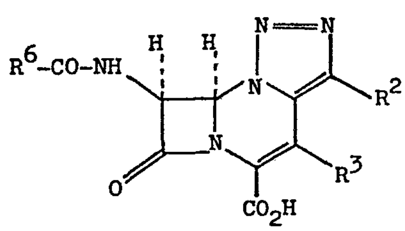

(II)

and salts thereof wherein $R^2$ and $R^3$ are as defined in relation to formula (I) and $R^6$ is a group such that $R^6$—CO—NH— is an acylamino group as found in antibacterially active penicillins or cephalosporins.

Suitable values for $R^2$ in the compounds of the formulae (I) and (II) include the hydrogen atom and methyl, ethyl, propyl, butyl, phenyl, p-nitrophenyl and p-methoxycarbonylphenyl. Favoured values for $R^2$ include the hydrogen atom and the methyl and ethyl groups. The preferred value for $R^2$ is the hydrogen atom.

Suitable values for $R^3$ in the compounds of the formulae (I) and (II) include the hydrogen atom and methyl, ethyl, propyl, butyl, acetoxymethyl and the like, such as those specifically described in the Examples herein.

Favoured values for $R^3$ include the methyl, ethyl, and acetoxymethyl group. The preferred value for $R^3$ is the methyl group.

From the foregoing it will be realised that certain particularly favoured antibacterially active compounds of this invention are those of the formula (III):

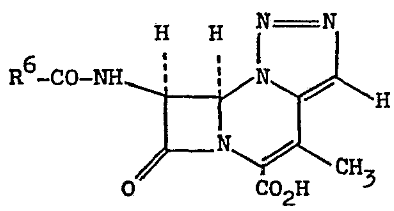

(III)

and salts thereof wherein $R^6$ is as defined in relation to formula (II).

Suitable groups $R^6CO$ for inclusion in the compounds of the formula (II) and (III) include those of the sub-formulae (a)—(d):

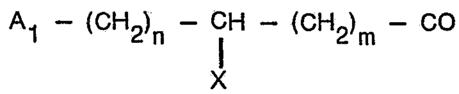

(a)

$$A_2 - CO$$ (b)

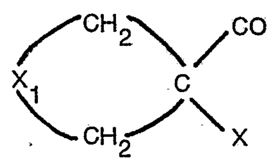

(c)

$$A_2 - X_2 - (CH_2)_n - CO$$ (d)

wherein n is 0, 1 or 2; m is 0, 1 or 2; $A_1$ is $C_1$—$C_6$ alkyl, $C_3$—$C_6$ cycloalkyl, cyclohexenyl, cyclohexadienyl, phenyl, hydroxy-phenyl, thienyl or pyridyl group; X is a hydrogen or halogen atom, a carboxylic acid, carboxylic ester, azido, tetrazolyl, hydroxy, acyloxy, amino, ureido, guanidino or acylureido group; $A_2$ is an aromatic group such as a phenyl, a 2,6-dimethoxyphenyl, 2-alkoxy-1-naphthyl, 3-arylisoxazolyl or 3-aryl-5-methylisoxazolyl group; $X_1$ is a $CH_2OCH_2$, $CH_2SCH_2$ or $(CH_2)_n$ group; $X_2$ is an oxygen or sulphur atom.

Favoured groups $R^6$ for inclusion in the compounds of the formulae (II) and (III) include those of the sub-formulae (e) and (f):

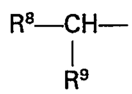

(e)

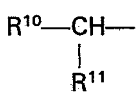

(f)

wherein $R^8$ is a phenyl, thienyl or phenoxy group; $R^9$ is a hydrogen atom or methyl group; $R^{10}$ is a phenyl, p-hydroxyphenyl, thienyl or cyclohexadienyl group; and $R^{11}$ is a hydroxyl, amino or carboxylic acid group or lower alkyl or pheny tolyl or indanyl ester thereof.

One apt group of the sub-formula (e) is the phenoxymethyl group. A second apt group of the sub-formula (e) is the benzyl group, a third apt group of the sub-formula (e) is the thienylmethyl group.

One suitable group of the formula $R^6CO$ is the D-phenylglycyl group.

Another suitable group of the formula $R^6CO$ is the D-p-hydroxyphenylglycyl group.

Another suitable group of the formula $R^6CO$ is the D-mandelyl group.

Another suitable group of the formula $R^6CO$ is the malonyl group.

Another suitable group of the formula $R^6CO$ is the benzoyl group.

Another suitable group of the formula $R^6CO$ is the 2-thienylacetyl group.

Another suitable group of the formula $R^6CO$ is the 3-thienylacetyl group.

Another suitable group of the formula $R^6CO$ is the 2-thienyl-$\alpha$-carboxyacetyl group.

Another suitable group of the formula $R^6CO$ is the 3-thienyl-$\alpha$-carboxyacetyl group.

Another suitable group of the formula $R^6CO$ is the phenoxyacetyl group.

One group of novel intermediates of this invention is that of the formula (IV):

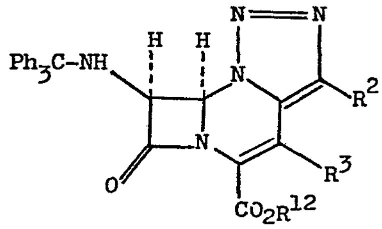

(IV)

wherein $R^2$ and $R^3$ are as defined in relation to formula (I) and $R^{12}$ is a group such that $CO_2R^{12}$ is an ester which is readily convertible to a carboxylic acid or a salt thereof.

Particularly apt values for $R^2$ and $R^3$ are those referred to in relation to formula (II).

A particularly favoured group $R^{12}$ is the tertiary butyl group.

A further particularly favoured group $R^{12}$ is the benzyl group.

A further group of novel intermediates of this invention is that of the formula (V):

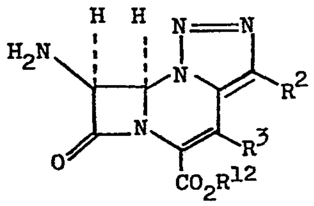

(V)

and salts thereof wherein $R^2$, $R^3$ and $R^{12}$ are as defined in relation to formula (IV).

The compounds of this invention may be in the form of an alkali metal, alkaline earth metal or nitrogenous base salt. Particularly suitable salts include the sodium, potassium, and ammonium. A preferred salt is the sodium salt.

The compounds of this invention which contain a basic group in the acylamino side chain can also form acid addition salts with pharmaceutically acceptable acids such as hydrochloric, or other strong acids. Most suitably those compounds which contain a basic group in the acylamino side chain are in zwitterionic form.

Intermediates of this invention may be esterified for example by a tertiary butyl group or like acid labile group or by a benzyl, p-nitrobenzyl or like hydrogenolysable group.

The antibacterial agents of this invention may be in the form of in-vivo hydrolysable esters.

Examples of suitable in vivo hydrolysable esters of the compounds of this invention include those which break down readily in the human body to leave the parent acid or its salt, for example acyloxylalkyl esters such as acetoxymethyl, pivaloyloxymethyl, $\alpha$-acetoxyethyl, $\alpha$-acetoxybenzyl, $\alpha$-pivaloyloxyethyl, $\alpha$-ethoxycarbonyloxyethyl esters and lactone esters such as the phthalidyl esters.

The present invention also provides antibacterial pharmaceutical compositions which comprise a compound of the formula (II) together with a pharmaceutically acceptable carrier.

The compositions of this invention are normally adapted for administration to humans. Such compositions may be formulated in a conventional manner for antibacterial agents, for example, in a similar manner to known penicillins or cephalosporins. Unit dose formulations according to this invention will normally contain from 100 mg to 4000 mg, more usually from 125 mg to 1000 mg and generally from 250 mg to 500 mg of a compound of the formula (II).

The compounds of this invention may be provided in orally administrable form, for example as tablets or capsules.

The compounds of this invention may be provided in a form suitable for administration by injection or infusion, for example in the form of a sterile salt such as the sterile sodium salt sealed in a vial or ampoule.

The compositions of this invention may be used to treat infections due to susceptible bacteria in humans and in domestic animals. Thus for example humans may be treated for diseases due to gram-positive micro-organisms such as *Staphylococcus aureus*. Similarly, the compositions may be used for the treatment of mastitis in cattle, for example by intramammary administration.

The compounds of this invention may be prepared according to the sequences outlined in Schemes 1—6:

Scheme 1

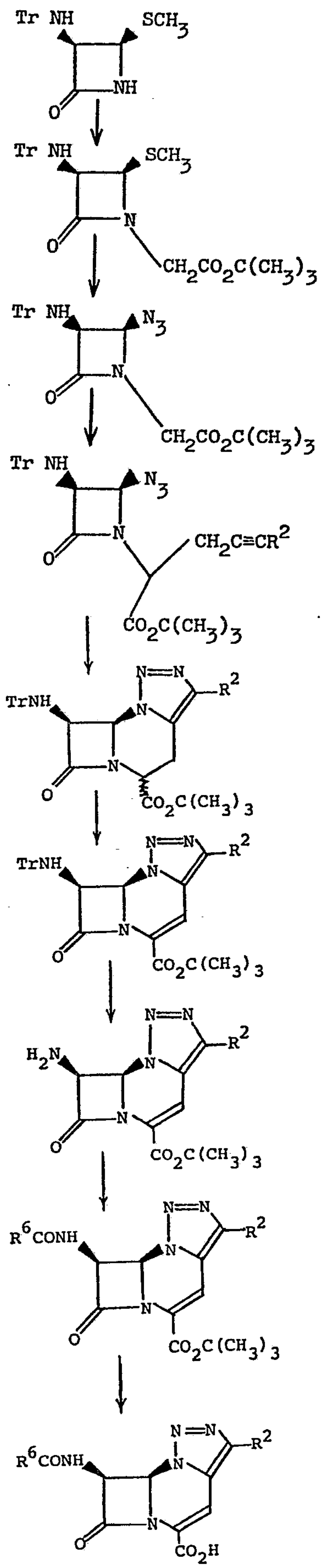

Scheme 2

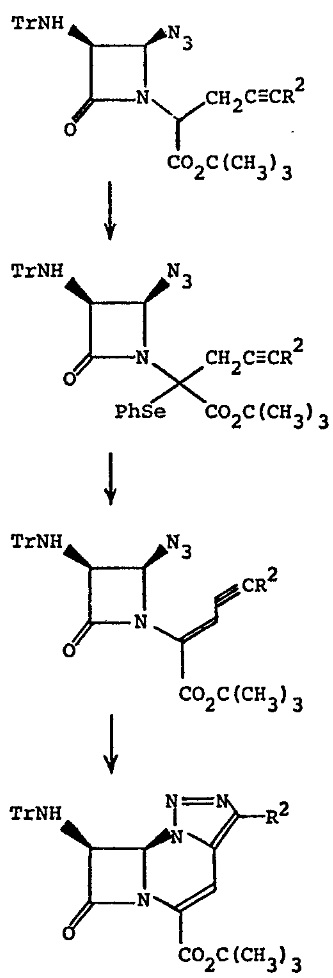

Scheme 3

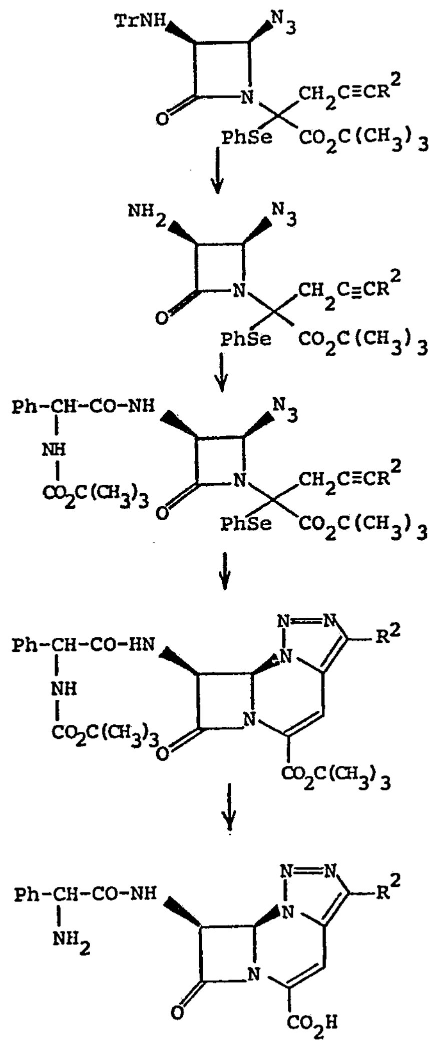

Scheme 4

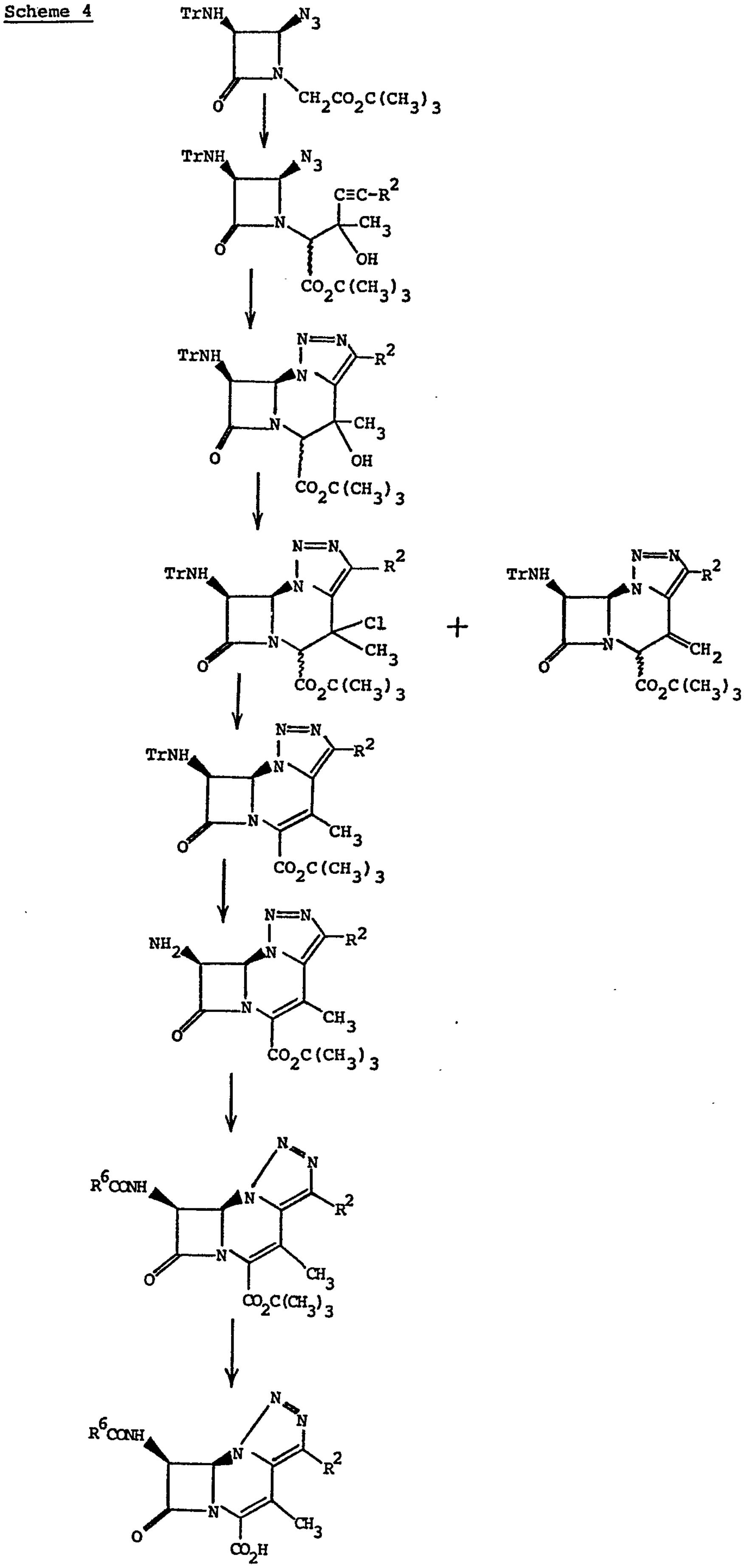

Scheme 5

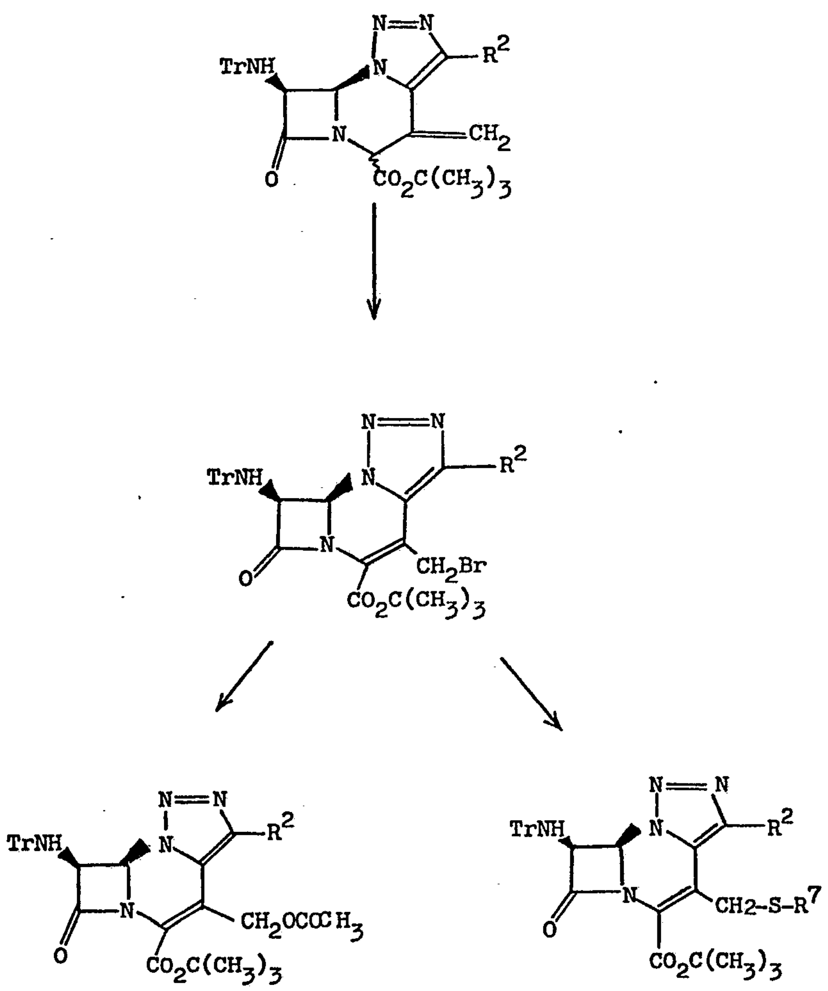

Scheme 6

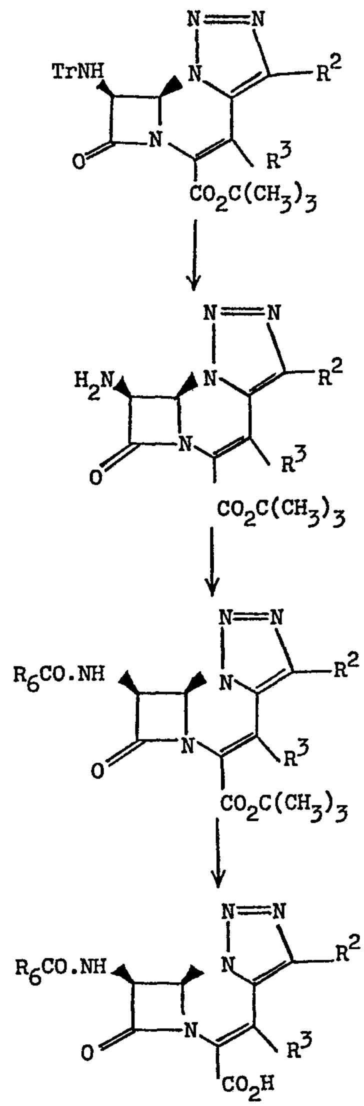

The present invention provides a process for the preparation of the compounds of the formula (II) as hereinbefore defined which process comprises the acylation of a corresponding compound of the formula (V) as hereinbefore defined with a reactive acylating derivative of a carboxylic acid of the formula (VI):

$$R^6—CO_2H \qquad (IV)$$

where in $R^6$ is as defined in relation to formula (II) and in which any acylatable group is optionally protected and thereafter performing one or more of the following reactions:

(a) removing any protecting group from the group $R^6$

(b) hydrolysing or hydrogenolysing the ester group $CO_2R^{12}$ to yield a free or salted carboxylic group.

The term "acylating derivative of a carboxylic acid" means any N-acylating compound known to be suitable for the performance of analogous reactions with 6-aminopenicillanic acid, 7-aminocephalosporanic acid, 7-aminodesacetoxycephalosporanic acid or salt, silyl derivative or ester thereof. Reactive groups present in such acylating agents may be protected in conventional manner as will be well understood by those skilled in the arts of preparing semi-synthetic penicillins or cephalosporins.

A particularly suitable group $R^{12}$ is the t-butyl group which may be removed by such conventional procedures as reaction with an anhydrous acid such as trifluoroacetic acid, for example at an approximately ambient temperature optionally in the presence of an inert solvent.

The intermediates of the formula (V) may be prepared by the replacement of the triphenylmethyl group from a corresponding compound of the formula (IV). In general this may be accomplished by mild acid hydrolysis at a depressed temperature, for example by the use of p-toluene-sulphonic acid at 0° to

−20°C in an organic solvent such as methylene chloride.

The preparation and reaction of the intermediates may be prepared by the general methods of the proceding reaction schemes and as described in the Examples hereinafter.

In a further aspect this invention provides the useful intermediates of the formula (VII):

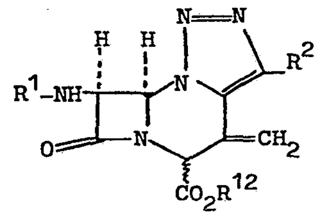

(VII)

wherein $R^1$ and $R^2$ are as defined in relation to formula (I) and $R^{12}$ is defined in relation to formula (V).

In such compounds $R^1$ is most suitably a trityl group; $R^2$ is a hydrogen atom and $R^{12}$ is a tertiary butyl group.

Reaction of a compound of the formula (VII) in an inert solvent at a depressed temperature with a non-nucleophilic base causes isomerisation to the corresponding compounds containing an endocyclic (as opposed to the exocyclic) double bond with a methyl group at the 8-position.

In yet a further aspect this invention provides the useful intermediates of the formula (VIII):

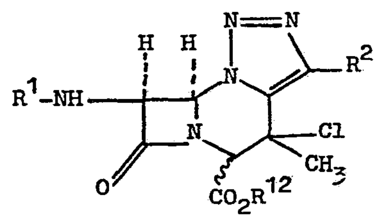

(VIII)

wherein $R^1$ and $R^2$ are as defined in relation to formula (I) and $R^{12}$ is as defined in relation to formula (I).

In such compounds $R^1$ is most suitably a trityl group; $R^2$ is a hydrogen atom; and $R^{12}$ is a tertiary butyl group.

Reaction of a compound of the formula (VIII) with a base as described in relation to reaction of a compound of the formula (VII), also leads to the preparation of compound possessing an endocyclic double bond and a methyl group at the 8-position.

The useful intermediates of the formula (VII) may be converted into the corresponding 8-bromomethyl compound by reaction with dibromodiethylmalonate in an aprotic solvent such as tetrahydrofuran at a depressed temperature such as −80° to −40°C. The resulting 8-bromomethyl compound may be used as an intermediate in conventional displacement reactions in which the bromine atom is replaced by the residue of an oxygen or sulphur nucleophile.

In the following examples, which serve to illustrate the present invention, all temperatures are given in degrees Centigrade.

Examples 1—4, 8, 9, 13, 14, 17, 19, 20 and 24—26 are preparations of useful intermediates.

The sequence below has been described in the literature (E. G. Brain, A. J. Eglington, J. H. C. Nayler, M. J. Pearson, and R. Southgate J. C. S. Perkin 1, 1976, 447). See also British Patent 1355330.

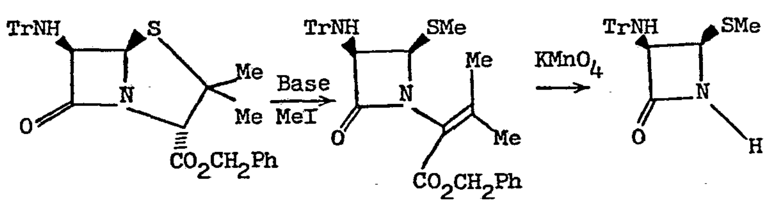


Example 1

t-Butyl 2-[(3R,4R)-4-methylthio-2-oxo-3-triphenylmethylaminoazetidin-1-yl]acetate (2)

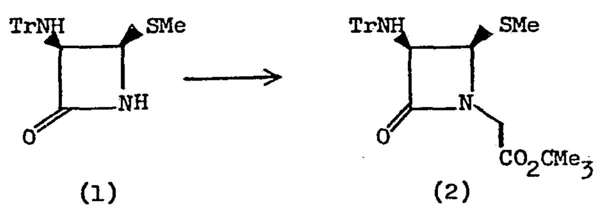

(1) (2)

(3R,4R)-4-Methylthio-3-triphenylmethylaminoacetidin-2-one [(1) ; 15g] was dissolved in dry dimethylformamide (225ml), containing *t*-butyl bromoacetate (9.24g) and powdered anhydrous potassium carbonate (9.9g). The mixture was vigorously stirred at room temperature for 20h., and then poured into ethyl acetate/brine. The organic layer was separated, washed successively with water and brine, dried and evaporated. Trituration with ether and filtration provided the product [(2) ; 10g]. Chromatography of the mother liquors on silica afforded further material (4.8g).m.p. 147—149° (ethyl acetate/petroleum ether). $[\alpha]_D^{23}$ −49.9° (c − 1 in chloroform). νmax (Nujol) 3300, 1785, 1740cm$^{-1}$. δ *ppm* ($CDCl_3$) 1.35 (9H, s), 1.65 (3H, s), 2.85 (1H, d, 7 Hz, exch), 3.4 and 4.05 (2H, $AB_q$, J 17 Hz), 4.11—4.58 (2H, m), 7.0—7.7 (15H, m). (*Found:* C, 71.2; H, 6.4; N, 5.7; S, 6.8; $C_{29}H_{32}N_2O_3S$ requires C, 71.3; H, 6.6; N, 5.7; S, 6.6%).

Example 2

t-Butyl 2-[3S,4R)-4-azido-2-oxo-3-triphenylmethylaminoazetidin-1-yl]-acetate (3) and t-butyl 2-[(3S,4S)-4-azido-2-oxo-3-triphenylmethylaminoazetidin-1-yl]-acetate (4)

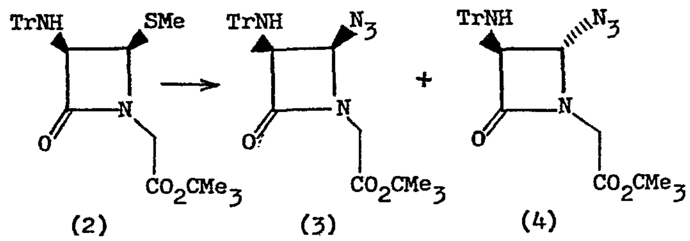

(2) (3) (4)

The lactam [(2) ; 8.48g] was dissolved in dry carbon tetrachloride (500ml) and the solution cooled to −20°. Chlorine (1.23g) in dry carbon tetrachloride (50ml) was added dropwise over 1h., and then the solution was allowed to warm to 0°. The solvent was evaporated, the residue dissolved in carbon tetrachloride (100ml) and the solvent re-evaporated. The product was dried (2mm Hg 2h.) and then dissolved in dry dimethylformamide (400ml). Powdered sodium azide (2.27g) was added, the mixture stirred at room temperature for 18h., and then poured into ethyl acetate/water. The organic layer was separated, washed with brine (x2), dried and evaporated. Chromatography on silica gave the *cis*-isomer [3 ; 6.11g). m.p. 142—143° (ethyl acetate/petroleum ether). $[\alpha]_D^{23} = +20.3$ (c = 1 in chloroform). νmax ($CHCl_3$) 2100, 1770, 1735cm$^{-1}$. δ *ppm* ($CDCl_3$) 1.45 (9H, s), 2.93 (1H, d, J 11 Hz, exch.), 3.57 and 4.22 (2H, ABq, J 18 Hz), 4.40—4.77 (2H, m, becomes 2H, s, at 3.95 exch.), 7.3—8.0 (15H, m). (*Found:* C, 69.7; H, 6.0; N, 14.5; $C_{28}H_{29}N_5O_3$ requires C, 69.6; H, 6.0; N, 14.5%).

Further elution gave the *trans*- isomer [4 ; 2.2g], m.p. 135° (ethyl acetate/petroleum ether). $[\alpha]_D^{23} = -52.8°$ (c=1.1 in chloroform). νmax ($CHCl_3$) 2075, 1770, 1735cm$^{-1}$. δ *ppm* ($CDCl_3$) 1.46 (9H, s), 2.90 (1H, d, J 10 Hz exch.),3.53 and 4.18 (2H, ABq, J 19 Hz), 4.13 (1H, s), 4.35 (1H, s) 7.1—7.8 (15H, m). (*Found:* C, 69.5; H, 6.1; N, 14.5; $C_{28}H_{29}N_5O_3$ requires C, 69.6; H, 6.0; N, 14.5%).

Example 3

(3S,4R)-4-Azido-1-(1-t-butoxycarbonyl-but-3-ynyl)-3-triphenylmethylaminoazetidin-2-one (5)

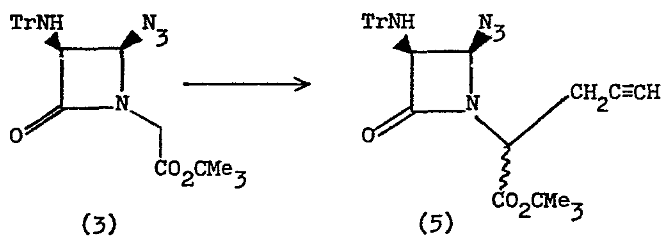

(3) (5)

To a stirred solution of hexamethyldisilazane (177mg) in dry tetrahydrofuran (2ml) under argon at 0° was added dropwise, n-butyl lithium (0.44ml ; 2.5M solution in hexane). After 10 min. the solution was cooled to −76° and the lactam [(3) ; 483mg] in dry tetrahydrofuran (5ml) added over 5 min. After a further 10 min. a solution of propargyl bromide (600mg) in dry tetrahydrofuran (3ml) was added dropwise over 5 min. The reaction mixture was stirred for a further 15 min. and was then poured into ethyl acetate/very dilute hydrochloric acid. The organic layer was separated, washed with brine (x2), dried and evaporated. Chromatography on silica afforded the product [(5) ; 350 mg]. νmax. ($CHCl_3$) 3270, 2100, 1770, 1735cm$^{-1}$. The nmr spectrum was complex but indicated that the product was a mixture of epimers.

Example 4

(3aR,4S) t-Butyl 4,5,7.8-tetrahydro-5-oxo-4-triphenylmethylamino-3aH-azeto-[1,2-a] v-triazolo [3,4-c] pyrimidine-7-carboxylate (6)

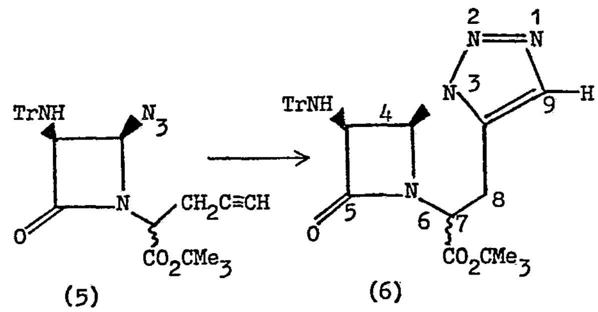

(5) (6)

The lactam [(5) ; 1.28g] was refluxed in dry toluene (750ml) for 8h. The solvent was evaporated and the residue chromatographed on silica to give two C—7 isomers of (6) as amorphous solids. Less polar isomer (tlc) (61%) $[\alpha]_D^{23} = -31°$ (c=1.09 in chloroform) νmax. 3315, 1783, 1738cm$^{-1}$. *δ ppm* ($CDCl_3$)1.32 (9H, s), 2.40 b (1H, s, exch.), 3.21 (1H, d, J 4 Hz), 4.5—4.75 (2H, m) 5.78 (1H, d, J 4 Hz), 7.1—7.7 (16H, m). (*Found:* C, 71.4; H, 6.1; N, 13.3; $C_{31}H_{31}N_5O_3$ requires C, 71.4; H, 6.0; N, 13.4%). More polar isomer (25%) $[\alpha]_D^{23} = -15°$ (c=1.13 in chloroform). νmax 3300, 1788, 1740cm$^{-1}$. *δ ppm* ($CDCl_3$) 1.49 (9H, s), 2.3 b (1H, s, exch.), 3.03—3.48 (2H, m), 3.56—3.8 (1H, m), 4.63 b (1H, s, becomes d, J 4 Hz on exch.), 5.38 (1H, d, J 4 Hz), 7.0—7.55 (16H, m). (*Found:* C, 71.9; H, 6.0; N, 13.3; $C_{31}H_{31}N_5O_3$ requires C, 71.4; H, 6.0; N, 13.4%).

Example 5

(3aR, 4S) t-Butyl 4,5-dihydro-5-oxo-4-triphenylmethylamino-3aH-azeto-[1.2-a] v-triazolo [3.4-c] pyrimidine-7-carboxylate (8)

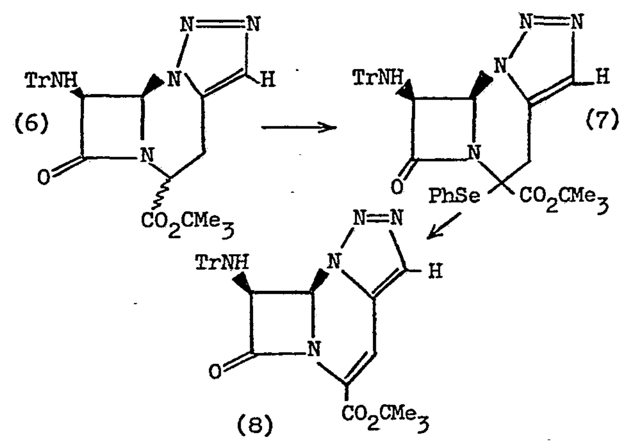

(6) (7) (8)

Hexamethyldisilazane (177mg) in dry tetrahydrofuran (3ml) was cooled to 0° under argon, and n-butyl lithium (0.44ml of 2.5 M in hexane) added. After 10 min. the stirred solution was cooled to −76° and a solution of the β-lactam [(6) ; 521mg, mixed isomers] in dry tetrahydrofuran (5 ml) was added dropwise over 10 min. After a further 10 min. phenylselenenyl bromide in dry tetrahydrofuran [0.8ml of a solution freshly prepared by addition of bromine (0.162ml) to diphenyl diselenide (0.94g) in tetrahydrofuran (4ml)] was added dropwise over 5 min. The solution was poured into ethyl acetate/brine and the organic layer separated, washed successively with dilute aqueous sodium bicarbonate and brine, dried and evaporated. Filtration through a short silica column gave the seleneno-triazole [(7) ; 448 mg] νmax ($CHCl_3$) 3360, 1795, 1725cm$^{-1}$.

The product[(7) ; 448mg] was dissolved in ethyl acetate (10ml) at 0° and *m*-chloroperbenzoic acid (226mg) added portionwise over 2 min. The solution was allowed to warm to room temperature and was then washed successively with aqueous sodium bicarbonate and brine, dried and evaporated. Chromatography on silica afforded the triazole [(8) ; 265mg] $[\alpha]_D^{23} = +1.12°$ (c = 1 in chloroform). νmax ($CHCl_3$) 3360, 1806, 1720, 1620cm$^{-1}$. *δ ppm* ($CDCl_3$) 1.51 (9H, s), 3.24 b (1H, s, exch), 5.09 b (2H, s, becomes sharp 2H, s, on exch.), 7.0—7.7 (16H, m), 7.78 (1H, s); λmax. (EtOH) 309 nm (ε = 8,700). (*Found:* C, 72.0; H, 5.9; N, 13.4; $C_{31}H_{29}N_5O_3$ requires C, 71.7; H, 5.6; N, 13.5%).

Example 6

(3aR, 4S) t-Butyl 4,5-dihydro-5-oxo-4-phenoxyacetamido-3aH-azeto-[1,2-a] v-triazolo [3,4-c]pyrimidine-7-carboxylate(10)

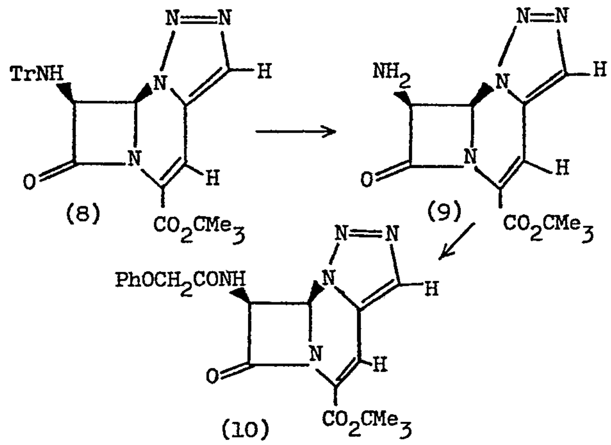

(8) (9) (10)

The lactam [(8) ; 100mg] was dissolved in dry methylene dichloride (3ml) at —20° and toluene-*p*-sulphonic acid (40mg) added dropwise in the minimum volume of methanol (0.5ml). After 2½ h. at + 5° the solvents were evaporated and the residue dried *in vacuo.*

The crude toluene-*p*-sulphonic acid salt of the free base (9) was dissolved in dry methylene chloride at —20° and triethylamine (80mg) added in methylene chloride (0.5ml), followed by phenoxyacetyl chloride (64mg) in methylene chloride (1ml). The reaction mixture was allowed to warm to ambient temperature and was then washed successively with aqueous sodium bicarbonate and brine (x2), dried and evaporated. Chromatography on silica gave the acylamino-derivative (10) as a white solid (71mg) by trituration of the purified product with ether. νmax (Nujol) 3315, 1805, 1718, 1680, 1625cm$^{-1}$. δ *ppm* ($CDCl_3$) 1.55 (9H, s), 4.37 (2H, s), 5.78 (1H, dd, J 4 Hz and 8 Hz). 5.89 (1H, d, J 4 Hz), 6.7—7.5 (7H, m), 7.77 (1H, s), λmax (EtOH) 313nm (ε = 9,600).

Example 7

(3aR,4S) 4,5-dihydro-5-oxo-4-phenoxyacetamido-3aH-azeto-[1,2-a] v-triazolo [3,4-c] pyrimidine-7-carboxylic acid (11)

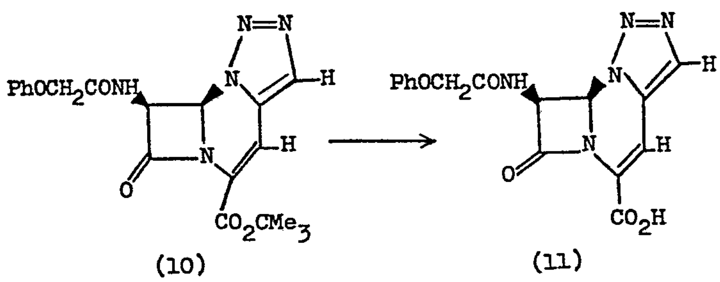

(10) (11)

The ester [(10) ; 47mg] was dissoved in trifluoroacetic acid (2ml) and the pale yellow solution left at room temperature for 35 min. The solvent was evaporated off, the residue treated with toluene, and the mixture evaporated; this procedure was repeated. Trituration with ether gave the free acid (11) as a white amorphous solid (36mg). νmax (Nujol) 3350 b, 1810, 1720sh, 1690, 1623cm$^{-1}$. δ *ppm* ($CDCl_3$ + 2 drops $(CD_3)_2SO$) 4.43 (2H, s), 5.2—5.7 b (1H, s, exch.), 5.95 (2H, m. collapses to two d's at 5.91 and 6.03, both J 4Hz, on exch.), 6.8—7.6 (6H, m), 7.88 (1H, s), 8.55 (1H, d, J 8 Hz, exch.). λmax (EtOH) 327nm (ε 6,500).

The minimum inhibitory concentrations (MIC) of this compound required to inhibit the growth of various bacteria on nutrient agar are tabulated below:—

| *Gram-positive bacteria* | *MIC (μg/ml)* |
|---|---|
| B. subtilis | 2.5 |
| Staph. aureus Oxford | 10 |
| Staph. aureus Russell* | 50 |
| β-Haemolytic Strep CN10 | 10 |

* β-Lactamase-producing strain

## Example 8

(3S,4R)-4-Azido-1-(1-t-butoxycarbonyl-4-phenyl-but-3-ynyl)-3-triphenyl-methylaminoazetidin-2-one (12)

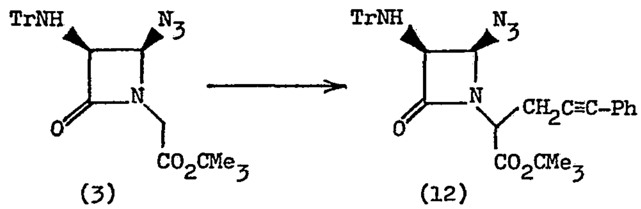

(3) (12)

The lactam [(3) ; 3.59g] was dissolved in tetrahydrofuran (100ml) and the solution cooled to −76° under argon. Lithium hexamethyldisilazide in tetrahydrofuran [15ml of a solution prepared by addition of n-butyl lithium (6.55ml ; 2.5M solution in hexane) to hexamethyldisilazane (2.63g) in tetrahydrofuran (20ml)] was added dropwise over 20 min. After a further 45 min. 3-phenyl-prop-2-ynyl bromide (1.6g) in tetrahydrofuran (5ml) was added dropwise over 10 min. The solution was stirred at −76° for 45 min. and then poured into ethyl acetate/water. The organic layer was separated, washed with brine, dried and evaporated. Chromatography on silica gave the product (12) as an amorphous solid (3.1g) $[\alpha]_D^{25.5} = -25.6°$ (c=1.57 in chloroform). $\nu$max ($CHCl_3$) 2125, 1770, 1740cm$^{-1}$. $\delta$ *ppm* ($CDCl_3$) 1.41 (9H, s), 2.75—3.0 (3H, m, 1H exch.) 4.35—4.8 (3H, m), 6.9—7.8 (20H, m).

## Example 9

(3aR,4S) t-Butyl 4,5,7,8-tetrahydro-5-oxo-9-phenyl-4-triphenylmethylamino-3aH-azeto-[1,2-a]triazolo [3,4-c]pyrimidine-7-carboxylate (13)

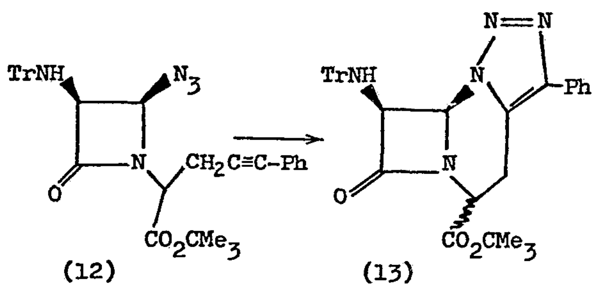

(12) (13)

The lactam [(12) ; 1.5g] was refluxed in toluene (500ml) under argon for 5.75h. The solvent was evaporated and the residue chromatographed on silica to give the phenyltriazole [(13) ; 1.374g] $[\alpha]_D^{25.5} = -5.7°$ (c=1 in chloroform) $\nu$max ($CHCl_3$) 3355, 1785, 1738cm$^{-1}$. $\delta$ *ppm* ($CDCl_3$) 1.29 (9H, s), 2.51 b (1H, s, exch.), 3.2—3.66 (2H, m), 4.68 (1H, t, J 3.5 Hz), 4.71 (1H, d, J 4 Hz), 5.72 (1H, d, J 4 Hz), 7.0—7.8 (20H, m). The product appeared to be a single C—7 isomer.

## Example 10

(3aR,4S) t-Butyl 4,5-dihydro-5-oxo-9-phenyl-4-triphenylmethylamino-3aH-azeto[1,2-a] v-triazolo [3,4-c]pyrimidine-7-carboxylate (15)

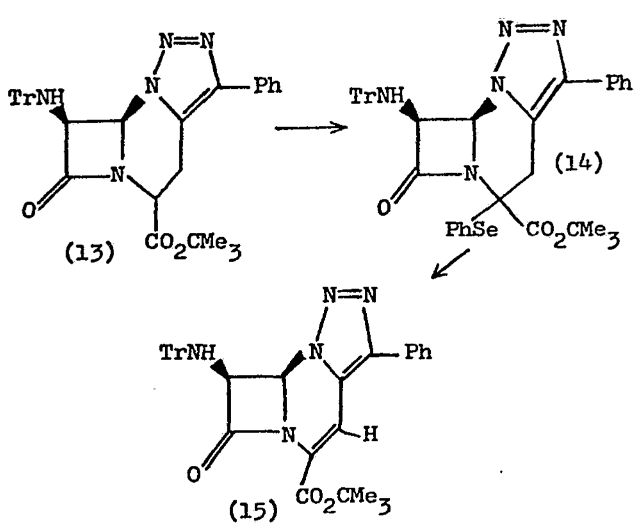

(13) (14) (15)

Hexamethyldisilazane (861mg) was dissolved in tetrahydrofuran (15ml) under argon at 0° and n-butyl lithium (2.14ml ; 2.5M solution in hexane) added. After 10 min. the solution was cooled to −76° and the lactam [(13) ; 1.452g] added dropwise in dry tetrahydrofuran (20ml) over 10 min. After a further 10 min. phenylselenenyl bromide in dry tetrahydrofuran [7.0ml of a solution freshly prepared by addition of bromine (0.324ml) to diphenyl diselenide (1.88g) in tetrahydrofuran (8ml)] was added dropwise over 15 min. The reaction mixture was poured into ethyl acetate, the organic layer washed successively with aqueous sodium bicarbonate, water, and brine, dried and evaporated. Chromatography on silica afforded the selenide [(14) ; 1.44g] $\nu$max ($CHCl_3$) 3350, 1792, 1722$cm^{-1}$. $\delta$ *ppm* ($CDCl_3$) 1.49 (9H, s) 2.47 b (1H, s, exch.). 3.0 and 3.56 (2H, ABq, J 18 Hz), 4.69 b (1H, s, becomes d J4 Hz on exch.), 5.61 (1H, d, J 4 Hz), 6.8—7.6 (25H, m).

The ester [(14) ; 1.37g] was dissolved in ethyl acetate (60ml) at 0° and m-chloroperbenzoic acid (628mg) added portionwise over 10 min. After 1h. at 0° the mixture was washed successively with dilute aqueous sodium bicarbonate and brine, dried and evaporated. Silica-gel chromatography gave the required product [(15) ; 932mg) $[\alpha]_D^{23} = 172.6°$ (c=1.37 in chloroform) $\nu$max. ($CHCl_3$) 3355, 1805, 1715, 1605$cm^{-1}$. $\delta$ *ppm* ($CDCl_3$) 1.52 (9H,s), 3.3 b (1H, s, exch.), 5.1 (2H, d, collapses to s on exch.) 6.8—7.8 (21H, m). $\lambda$max. (EtOH) 333nm ($\varepsilon$ 17,900) (*Found:* C, 74.7; H, 5.8; N, 11.7. $C_{37}H_{33}N_5O_3$ requires C, 74.6; H, 5.5; N, 11.8%).

Example 11

(3aR,4S) t-Butyl 4,5-dihydro-5-oxo-9-phenyl-4-D-mandelylamino-3aH-azeto-[1,2-a] v-triazolo [3,4-c] pyrimidine-7-carboxylate (17)

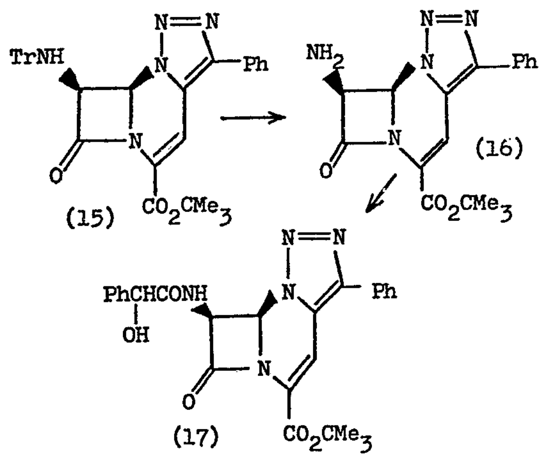

(15) (16) (17)

The lactam [(15) ; 298mg] was dissolved in dry methylene chloride (10ml) and the solution cooled to −20°. Toluene-p-sulphonic acid (209mg) was added dropwise in the minimum volume of methanol and the solution allowed to warm to room temperature. After 1 hr. the solvent was evaporated and the residue dissolved in ethyl acetate. The solution was washed with dilute aqueous sodium bicarbonate, followed by brine, dried and evaporated. The free base (16), a white solid, was dried *in vacuo.*

The total crude product (16) was dissolved in dry methylene chloride (10ml) and the solution cooled to −20°. D-Mandelyl O-carboxyanhydride (196mg) was added in one portion. The solvent was evaporated and the residue dissolved in ethyl acetate. The solution was washed successively with aqueous sodium bicarbonate, water, and brine, dried and evaporated. Chromatography on silica gave the acylamino-derivative [(17) ; 208mg] $[\alpha]_D^{23} = +63.85°$ (c = 1, in chloroform). $\nu$max. 3380, 3300 b, 1810, 1712, 1690sh $cm^{-1}$. $\delta$ *ppm* ($CDCl_3$) 1.57 (9H, s), 5.35 (1H, s), 5.88 (1H, d, J4 Hz), 6.03 (1H, dd, J 4 Hz and 9 Hz), 7.0—7.5 (11H, m), 8.02 (1H, d, J 9 Hz). $\lambda$max. (EtOH) 337nm ($\varepsilon$ 12,800).

Example 12

(3aR, 4S) 4,5-Dihydro-5-oxo-9-phenyl-4-D-mandelylamino-3aH-azeto-[1,2-a] v-triazolo [3,4-c]-pyrimidine-7-carboxylic acid (18)

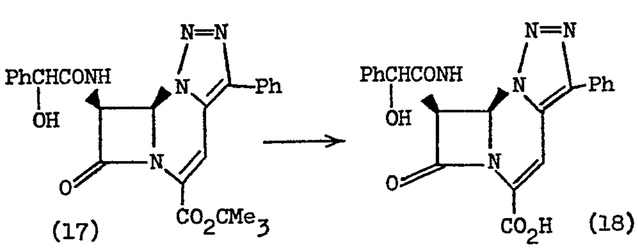

(17) (18)

The ester [(17) ; 100mg] was dissolved in trifluoroacetic acid (2ml). After 30 min. at room temperature the solvent was evaporated off, the residue treated with toluene, and the mixture evaporated; this procedure was repeated. Trituration with ether gave the free acid (18) as a white amorphous solid (82mg). $[\alpha]_D^{22} = 109°$ (c = 0.76 in aqueous sodium bicarbonate) $\nu$max (KBr) 3400 b, 1790, 1720, 1670 $cm^{-1}$. $\delta$ *ppm* ($D_6$DMSO) 4.86 (1H, s), 5.0 b (2H, s, exch.), 5.85 (1H, dd, J 4 Hz and 9 Hz), 6.1 (1H, d, J 4 Hz), 7.0—7.8 (11H, m), 8.91 (1H, d, J 9 Hz). $\lambda$max [80% EtOH + 20% (0.3% w/w sodium bicarbonate)] 266nm ($\varepsilon$ 9,700), 404 ($\varepsilon$ 5,570).

The minimum inhibitory concentrations (MIC) of this compound required to inhibit the growth of various bacteria in nutrient broth are tabulated below. ($10^{-4}$ dilution).

| *Gram-positive bacteria* | *MIC (μg/ml)* |
|---|---|
| Staph. aureus Oxford | 31 |
| Staph. aureus Russell* | 125 |

*β-Lactamase producing strains

| *Gram-negative bacteria* | *MIC (μg/ml)* |
|---|---|
| E. coli JT 1 | 125 |
| E. coli NCTC 10418 | 125 |

Example 13

(3S,4R) - 4 - Azido - 1 - (1 - t - butoxycarbonyl - 1 - phenylselenenyl - 4 - phenyl - but - 3 - ynyl) - 3 - triphenylmethylaminoazetidin - 2 - one (19)

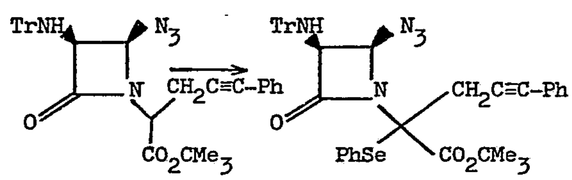

(12) (19)

Hexamethyldisilazane (624mg) in tetrahydrofuran (5ml) was cooled to 0° under argon and n-butyl lithium (1.56ml; 2.5M solution in hexane) added. After 15 min. the solution was cooled to −76° and the lactam [(12) ; 1.41g] in tetrahydrofuran (20ml) added dropwise over 15 min. After 10 min phenylselenenyl bromine in tetrahydrofuran [3.1 ml of solution prepared by adding bromine (0.162g) to diphenyl diselenide (0.94g) in tetrahydrofuran (5ml)] was added over 5 min. The reaction mixture was poured into ethyl acetate/water, the organic layer separated, washed with brine (x2), dried, and evaporated. Silica gel chromatography gave [(19) ; 1.48g] $\nu$max. ($CHCl_3$) 3340, 2125, 1765, 1730 $cm^{-1}$. $\delta$ *ppm* (major isomer) (*ca* 85%) 1.48 (9H, s), 2.72 b (1H, s, exch.), 3.07 and 3.89 (2H, ABq, J 16 Hz) 3.43 b (1H, s, becomes d J 4 Hz on exch), 4.19 (1H, d, J 4 Hz), 6.8—7.6 (25H, m). A singlet at 1.38$\delta$ was probably due to the —$CO_2C$*$Me_3$* of the minor isomer. A doublet (J 4 Hz) at 4.67$\delta$ being one of the β-lactam protons of the minor isomer.

Example 14

(3S, 4R) - 4 - Azido - 1 - (1 - 1t - butoxycarbonyl - 1 - phenylselenenyl - 4 - phenyl - but - 3 - ynyl) - 3 - (N - t - butoxycarbonyl - D - α - phenylglycyl)aminoazetidin - 2 - one (21)

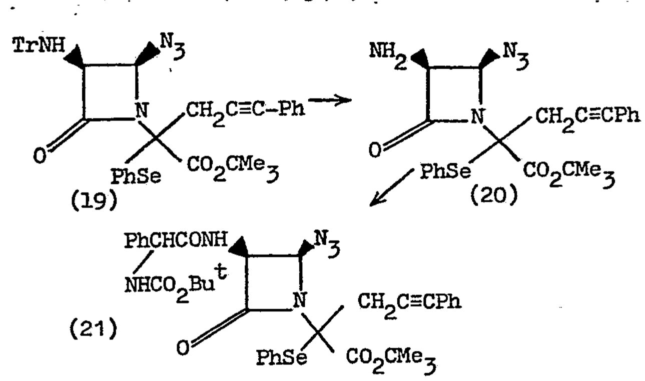

The lactam [(19) ; 753mg] was dissolved in methylene chloride (10ml) at —20° and toluene-*p*-sulphonic acid (209mg) in the minimum volume of methanol added dropwise over 2 min. The reaction mixture was kept at —5° for 16h and then at +3° for 3h. The solvents were evaporated off and the residue dissolved in ethyl acetate. The solution was washed successively with dilute aqueous sodium bicarbonate and brine (x2), dried and evaporated. The residual gum (20) was dried *in vacuo.*

Methyl chloroformate (104mg) in dry tetrahydrofuran (6ml) was cooled to —20°. To this solution was added, dropwise with stirring, a solution of N-(t-butoxycarbonyl)-D-$\alpha$-phenylglycine (276mg), triethylamine (112mg), and N,N-dimethylbenzylamine (1 drop) in tetrahydrofuran (10ml) over 5 min. After a further 30 min at —20° the crude free base (20) was added dropwise over 5—10 min in dry tetrahydrofuran (10 ml). After stirring for a further 1h. the mixture was poured into ethyl acetate/water. The organic layer was separated, washed successively with very dilute hydrochloric acid and brine (x2) dried and evaporated. Chromatography on silica provided the product [(21) ; 500mg]. $\nu$max. 3440, 2135, 1780, 1710 b $cm^{-1}$.

Example 15

(3aR,4S)t - Butyl 4,5 - dihydro - 5 - oxo - 9 - phenyl - 4 - (N - t - butoxycarbonyl - D - $\alpha$ - phenylglycyl)amino - 3aH - azeto[1,2 - a]v - triazolo[3,4 - c]pyrimidine - 7 - carboxylate (23)

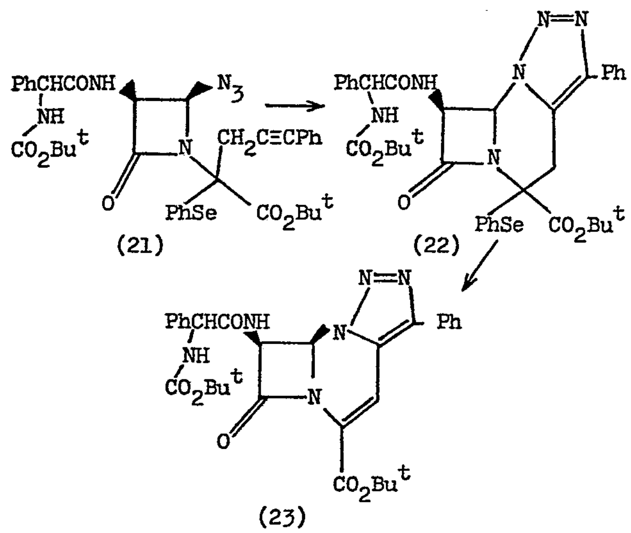

(21) (22) (23)

The lactam [(21) ; 457mg] was refluxed in dry toluene (175ml) under argon for 11½h. The solvent was evaporated and the residue (22) dissolved in ethyl acetate and the solution cooled to 0°. m-Chloroperbenzoic acid (212mg) was added in portions over 1—2 min and the mixture allowed to warm to room temperature. The solution was washed successively with aqueous sodium bicarbonate and brine (x2), dried and evaporated. Chromatography on silica afforded the required product [(23) ; 251mg] $[\alpha]_D^{23} = +44.63°$ (c = 1 in chloroform) $\nu$max ($CHCl_3$) 3380, 3270, 1808, 1710 b $cm^{-1}$. $\delta$ *ppm* ($CDCl_3$) 1.3 (9H,s), 1.54 (9H, s), 4.55 (1H, s, exch.), 5.35 (1H, m, becomes s on exch), 5.67 (1H, dd, J 9 Hz, 4 Hz), 5.87 (1H, d, J 4 Hz), 7.0—8.0 (12H, m) $\lambda$max (EtOH) 337nm ($\varepsilon$ 12,900), 254nm ($\varepsilon$ 8,100).

Example 16

(3aR,4S)-4,5-Dihydro-5-oxo-9-phenyl-4-D-$\alpha$-phenylglycylamino-3aH-azeto [1,2-a] v-triazolo [3,4-c]pyrimidine-7-carboxylic acid, trifluoroacetic acid salt (24)

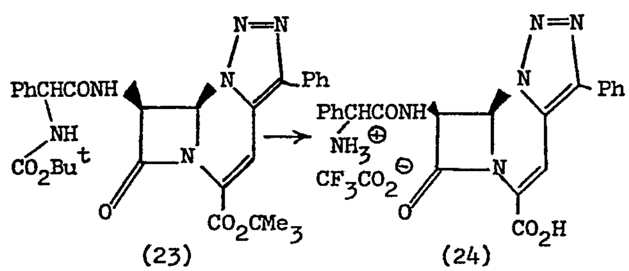

(23) (24)

The lactam (23) (95mg) was dissolved in trifluoroacetic acid (2ml). After 30 min at room temperature the solvent was evaporated off, the residue treated with toluene, and the mixture evaporated; this procedure was repeated. Trituration with ether gave the TFA salt [(24) ; 69mg]

$[\alpha]_D^{22}$ + 43.6° (c = 0.32 in DMSO) νmax (KBr) 3400 b, 3050 b, 1795, 1670 b cm$^{-1}$. δ *ppm* ($D_6$—DMSO) 3.0—5.0 b (4H, s, exch.), 4.94 (1H, s), 6.1 (2H, m becomes 2 d's at 5.85 and 6.12, both J 4 Hz, on exch.), 7.1—7.8 (11H, m), 9.25 (1H, d, J 9 Hz, exch.). λmax (EtOH) 327nm (ε 12,000), 260nm (ε 9,200).

The minimum inhibitory concentrations (MIC) of this compound required to inhibit the growth of various bacteria in nutrient broth are tabulated below:— ($10^{-4}$ dilution).

| *Gram-positive bacteria* | *MIC (μg/ml)* |
|---|---|
| Staph aureus Oxford | 62 |
| Staph aureus Russell* | 125 |
| *β-Lactamase producing strain | |

| *Gram-negative bacteria* | *MIC (μg/ml)* |
|---|---|
| E. coli JT1 | 125 |
| E. coli NCTC 10418 | 125 |

Example 17

(3S,4R) - 4 - Azido - 1 - Z(1 - t - butoxycarbonyl - 4 - phenyl - but - 1 - en - 3 - ynyl) - 3 - triphenylmethylaminoazetidin - 2 - one (25)

(3S,4R,) - 4 - Azido - 1 - E(1 - t - butoxycarbonyl - 4 - phenyl - but - 1 - en - 3 - ynyl) - 3 - triphenylmethylaminoazetidin - 2 - one (26)

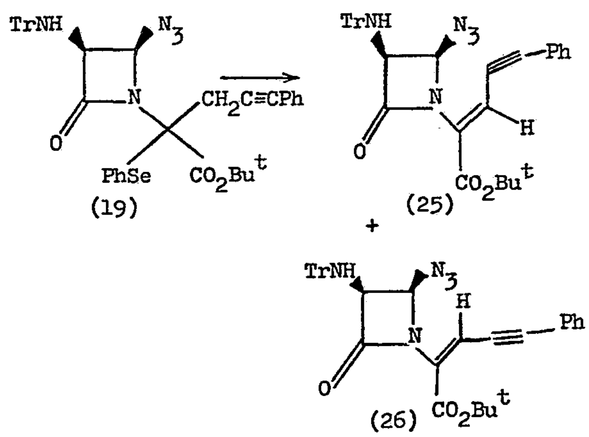
(19) (25) + (26)

The lactam [(19) ; 570mg] was dissolved in ethyl acetate (30ml) at 0° and m-chloroperbenzoic acid (260mg) added in portions over 5 min. The mixture was washed successively with aqueous sodium bicarbonate, brine, dried, and evaporated. Chromatography gave the two isomers of the ene-yne.

Major (Z)-isomer (25) (60%). νmax ($CHCl_3$) 3340, 2205, 2125, 1785, 1715, 1608cm$^{-1}$ δ *ppm* ($CDCl_3$) 1.48 (9H, s), 2.96 b (1H, s, exch.), 4.47 b (1H, s, becomes d, J 4 Hz on exch.), 4.95 (1H, d, J 4 Hz), 6.49 (1H, s), 7.0—7.6 (20H m). Minor (E)-isomer (26) (20%) νmax 3350, 2190 (w), 2150, 1770, 1710cm$^{-1}$. δ *ppm* ($CDCl_3$) 1.48 (9H, s), 2.95 b (1H, s, exch.), 4.45 b (1H, s, becomes d, J 4 Hz, on exch.), 4.96 (1H, d, J 4 Hz), 7.02 (1H, s), 7.1—7.6 (20H, m).

Example 18

(3aR,4S) t-Butyl-4,5-dihydro-5-oxo-9-phenyl-4-triphenylmethylamino-3aH-azeto [1,2-a] v-triazolo [3,4-c] pyrimidine-7-carboxylate (15)

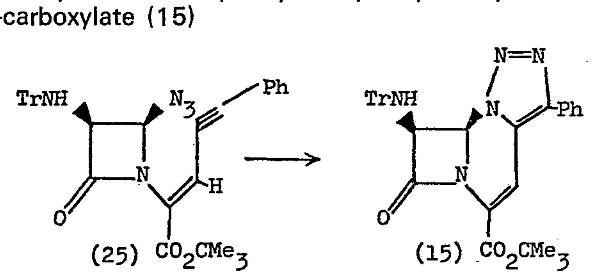
(25) (15)

The Z-isomer [(25) ; 245mg] was refluxed under argon in toluene (120ml) for 4h. The solvent was evaporated and the residue chromatographed on silica to give the product [(15) ; 219mg], identical in all respects to that prepared in Example 10.

The lactam [(26) ; 70mg] slowly decomposed when refluxed under argon in toluene (40ml).

Example 19

(3S,4R) - 4 - Azido - 1 - (1 - t - butoxycarbonyl - 4 - p - nitrophenyl - but - 3 - ynyl) - 3 - triphenylmethylaminoazetidin - 2 - one (27)

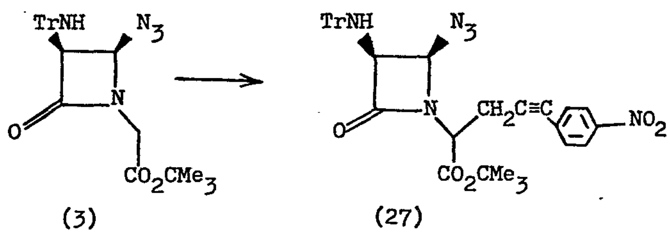

(3) (27)

Hexamethyldisilazane (975mg) was dissolved in dry tetrahydrofuran (20ml) at 0° under argon, and n-butyl lithium (2.42ml ; 2.5M solution in hexane) added. The solution was cooled to –76° after 10 min. and the lactam [(3) ; 2.6g] added dropwise in dry tetrahydrofuran (40ml) over 15 min. After a further 10 min 3-*p*-nitrophenyl-prop-2-ynyl bromide (1.454g) in dry tetrahydrofuran (10ml) was added dropwise over 10 min. After 1 h at –76° the mixture was poured into ethyl acetate/very dilute hydrochloric acid. The organic layer was separated, washed with brine (x2), dried, and evaporated. Chromatography on silica afforded the acetylenic lactam [(27) ; 1.004g] $[\alpha]_D^{23} = -19.96°$ (c = 1.05 in chloroform) $\nu$max ($CHCl_3$) 2125, 1775, 1740, 1600, 1520, 1345 $cm^{-1}$. $\delta$ *ppm* ($CDCl_3$)1.42 (9H, s), 2.75—3.15 (3H, m becomes 2H, d, at 2.95 J 6 Hz on exch.), 4.3—4.85 (3H, m), 7.05—8.35 (19H, m).

Example 20

(3aR,4S) t - Butyl 4,5,7,8-tetrahydro - 5 - oxo - 9 - p - nitrophenyl - 4 - triphenyl - methylamino - 3aH - azeto[1,2 - a]v - triazolo[3,4 - c]pyrimidine - 7 - carboxylate (28)

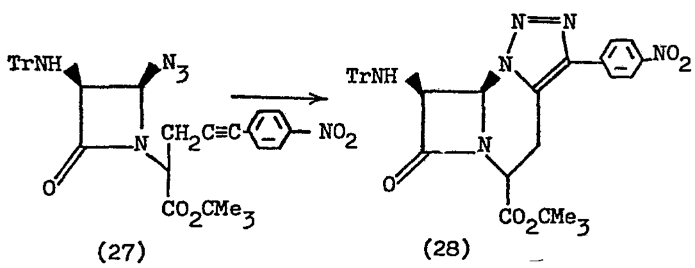

(27) (28)

The lactam [(27) ; 1.2g] was refluxed under argon in toluene (600ml) for 7h. The solvent was evaporated and the residue chromatographed on silica to give the triazole [(28) ; 1.08g]. $[\alpha]_D^{23} = +33.96°$ (c = 1.03 in chloroform). $\nu$max ($CHCl_3$) 1790, 1740, 1600, 1520, 1345$cm^{-1}$. $\delta$ *ppm* ($CDCl_3$) 1.29 (9H, s), 2.45 (1H, d, J 9 Hz exch.), 3.35—3.55 (2H, m), 4.65—4.9 (2H, m), 5.73 (1H, d, J 4 Hz), 7.0—7.6 (15H, m), 7.84 and 8.25 (4H, ABq, J 9 Hz).

Example 21

(3aR,4S) t - Butyl 4,5 - dihydro - 5 - oxo - 9 - p - nitrophenyl - 4 - triphenylmethyl - amino - 3aH - azeto [1,2 - a] v - triazolo[3,4 - c]pyrimidine - 7 - carboxylate (30)

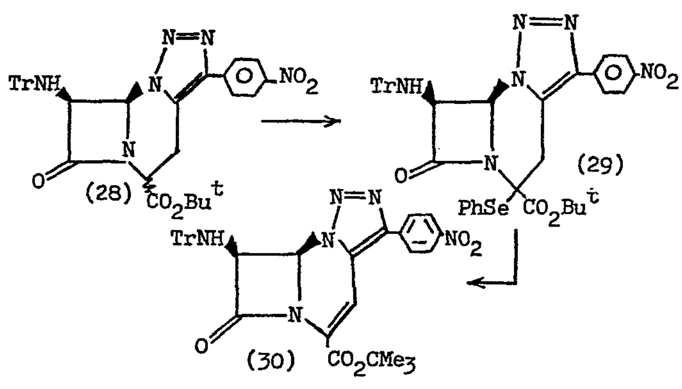

(28) (29) (30)

Hexamethyldisilazane (276mg) was dissolved in dry tetrahydrofuran (3ml) under argon at 0° and n-butyl lithium (0.68ml : 2.5M solution in hexane) added. The solution was cooled to −76° after 10 min and the lactam [(28) ; 1.0g] in tetrahydrofuran (8ml) added dropwise over 5 min. After 10 min phenylselenenyl bromide in tetrahydrofuran [1.25ml of a solution prepared by addition of bromine (0.162ml) to diphenyl diselenide (0.94g) in tetrahydrofuran (4ml)] was added dropwise over 3—4 min. The mixture was poured into ethyl acetate/very dilute hydrochloric acid and the organic layer separated, washed with brine, dried and evaporated. Filtration through a silica column provided the selenide [(29) ; 750mg]. The latter was dissolved in ethyl acetate (50ml) at 0° and m-chloroperbenzoic acid (330mg) added portionwise over 5 min. The reaction mixture was allowed to warm to room temperature and was then washed successively with dilute aqueous sodium bicarbonate, brine, dried, and evaporated. Chromatography afforded the required product [(30) ; 473mg]. m.p. 205—208° (ethyl acetate/petrol) $[\alpha]_D^{23} = 215.4°$ (c = 1 in chloroform) $\nu$max ($CHCl_3$) 1810, 1720, 1600, 1520, 1345cm$^{-1}$. $\delta$ *ppm* ($CDCl_3$) 1.56 (9H, s), 3.30 (1H, d, J 9 Hz exch.), 5.1—5.4 (2H, m, collapses to s 5.24 on exch.), 7.1—7.8 (16H, m), 7.96 and 8.38 (4H, ABq, J 9 Hz) $\lambda$max (EtOH) 344nm ($\varepsilon$ 18,100), 290nm ($\varepsilon$ 9,200) (*Found:* C, 69.2; H, 5.0; N, 13.0. $C_{37}H_{32}N_6O_5$ requires C, 69.4; H, 5.0; N, 13.1%).

Example 22

(3aR,4S) t-Butyl 4,5-dihydro-5-oxo-9-p-nitrophenyl-4-phenoxyacetamido-3aH-azeto [1,2-a] v-triazolo [3,4-c] pyrimidine-7-carboxylate (32)

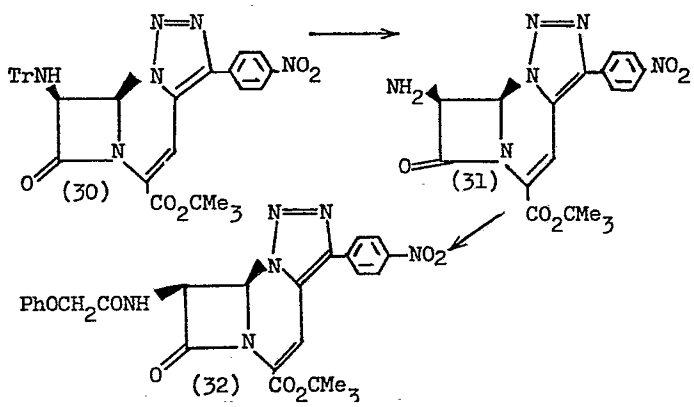

(30) (31) (32)

The lactam [(30) ; 320mg] was dissolved in dry methylene chloride (4ml) at −20° and toluene-*p*-sulphonic acid (104mg) added dropwise in the minimum volume of methanol. The reaction mixture was allowed to warm to room temperature and after 1h. the solvents were evaporated off. The residue was dissolved in ethyl acetate and the solution was washed successively with aqueous sodium bicarbonate and brine, dried and evaporated. The crude free base (31) was dried *in vacuo* and then dissolved in methylene chloride (7ml) at −20°. Triethylamine (56mg) in methylene chloride (1 ml) was added, followed by a solution of phenoxyacetyl chloride in methylene chloride (1 ml). The solution was allowed to warm to room temperature and was washed with water, followed by brine, dried and evaporated. Silica gel chromatography gave the acylamino-derivative [(32) ; 178mg] $[\alpha]_D^{23} = +184.2°$ (c = 1.03 in chloroform) $\nu$max (KBr) 3350, 1810, 1700, 1520, 1345cm$^{-1}$. $\delta$ *ppm* ($D_6$DMSO) 1.54 (9H, s), 4.38 (2H, s), 5.92 (1H, dd, J 9 Hz, 4 Hz), 6.19 (1H, d, J 4 Hz), 6.7—7.35 (5H, m), 7.4 (1H, s), 8.03 and 8.30 (4H, ABq, J 9 Hz), 9.00 (1H, d, J 9 Hz). $\lambda$max (EtOH) 346nm ($\varepsilon$ 18,850), 290nm ($\varepsilon$ 9,800).

Example 23

(3aR,4S) 4,5 - Dihydro - 5 - oxo - 9 - p - nitrophenyl - 4 - phenoxyacetamido - 3aH azeto[1,2 - a]v - triazolo[3,4 - c]pyrimidine - 7 - carboxylic acid (33)

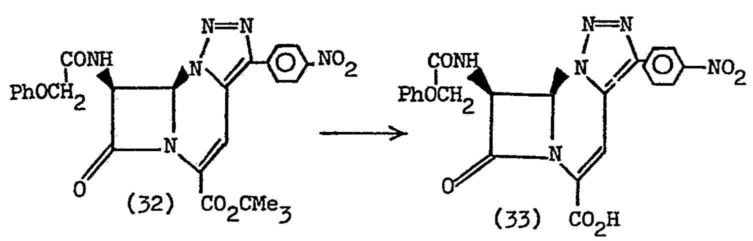

(32) (33)

The lactam [(32) ; 130mg] was dissolved in trifluoroacetic acid (4ml). After 30 min. the solvent was evaporated off, the residue treated with toluene, and the mixture evaporated; this procedure was repeated. Trituration with ether gave the free acid (33) as a yellow solid (96mg). $[\alpha]_D^{22} = 173.1$ (c = 1.04 in DMSO) $\nu$max (KBr) 3380, 1800, 1700, 1600, 1530, 1345cm$^{-1}$. $\delta$ *ppm* ($D_6$DMSO) 4.40

(2H, s), 5.8—6.05 (1H, m), 6.05—6.3 (1H, m), 6.7—7.6 (7H, m), 7.8—8.5 (4H, m), 9.01 (1H, d, J 9 Hz).

The minimum inhibitory concentrations (MIC) of this compound required to inhibit the growth of various bacteria in nutrient broth are tabulated below:

| *Gram-negative bacteria* | *MIC (μg/ml⁻¹)* |
|---|---|
| E.coli JT 1 | 500 |
| E. coli NCTC 10418 | 500 |

| *Gram positive bacteria* | *MIC (μg/ml⁻¹)* |
|---|---|
| Staph. aureus Oxford | 16 |
| Staph. aureus Russell* | 31 |

*β-lactamase producing strain

## Example 24

(3S,4R) - 4 - Azido - 1 - ( - 1 - t - butoxycarbonyl - 2 - hydroxy - 2 - methyl - but - 3 - ynyl) - 3 - triphenylmethylaminoazetidin - 2 - one (34)

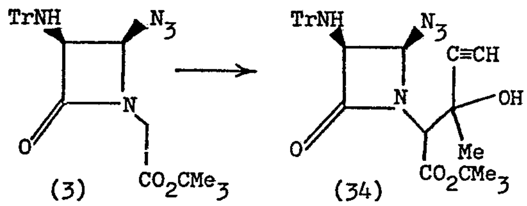

(3) (34)

Hexamethyldisilazane (0.88g) was dissolved in dry tetrahydrofuran at 0° under argon and n-butyl lithium (2.2ml ; 2.5M solution in hexane) added. The solution was stirred for 10 min. and then cooled to −76° and the lactam [(3) ; 2.42g] in tetrahydrofuran (30ml) added dropwise over 20—25 min. A further 20 min. later 3-butyn-2-one (400mg) in tetrahydrofuran (10ml) was added dropwise over 10 min. The reaction mixture was poured into ethyl acetate/very dilute hydrochloric acid and the organic layer separated. the latter was washed with brine (x2), dried and evaporated. Chromatography gave the amorphous product [(34) ; 2.325g]. νmax 3500, 3310, 1770, 1730cm$^{-1}$. (*Found:* C, 69.3; H, 5.9; N, 12.3. $C_{32}H_{33}N_5O_4$ requires C, 69.7; H, 6.0; N, 12.7%).

## Example 25

(3aR,4S) t - Butyl 4,5 - dihydro - 5 - oxo - 7H - 8 - hydroxy - 8 - methyl - 4 - triphenyl - methylamino - 3aH - azeto[1,2 - a] v - triazolo [3,4 - c]pyrimidine - 7 - carboxylate (35)

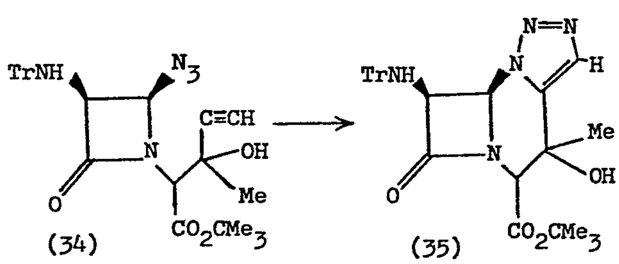

(34) (35)

The lactam [(34) ; 2.265g] was refluxed, under argon, in dry toluene (300ml) for 2h. The solvent was evaporated and the residue chromatographed on silica. Recrystallisation from benzene/petrol afforded the product [(35) ; 1.072g] m.p. 165—167° $[\alpha]_D^{23} = -36.9°$ (c = 0.78 in chloroform) νmax ($CHCl_3$) 3350, 1780, 1718cm$^{-1}$. *δ ppm* ($CDCl_3$)1.38 (9H, s), 1.69 (3H, s), 2.65 (1H, d, J 9 Hz exch), 3.66 (1H, s, exch), 4.45 (1H, s), 4.77 (1H, dd, J 4 Hz and 9 Hz, collapses to d J 4 Hz on exch), 5.60 (1H, d, J 4 Hz), 7.1—7.7 (15H, m), 7.81 (1H, s). (*Found:* C, 69.4; H, 6.1; N, 12.6. $C_{32}H_{33}N_5O_4$ requires C, 69.7; H, 6.0; N, 12.7%).

## Example 26

(3aR,4S) t-Butyl 4,5 - dihydro - 5 - oxo - 7H - 8 - chloro - 8 - methyl - 4 - triphenyl -

methylamino - 3aH azeto[1,2 - a] v-triazolo[3,4 - c]pyrimidine - 7 - carboxylate (36)
(3aR,4S) t - Butyl 4,5 - dihydro - 5 - oxo - 7H - 8 - methylene - 4 - triphenylmethyl - amino - 3aH azeto[1,2 - a]v - triazolo[3,4 - c]pyrimidine - 7 - carboxylate (37)

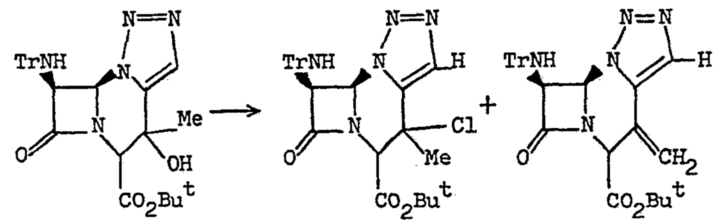

(35) (36) (37)

The lactam [(35) ; crystalline); 1.102g] was dissolved in dry tetrahydrofuran (50ml) and the solution cooled to −20°. Dry lutidine (0.51ml) was added followed by thionyl chloride (0.315ml) dropwise in tetrahydrofuran (5ml). After 5 min the precipitate was removed and the filtrate evaporated and redissolved in ethyl acetate. The solution was washed successively with very dilute hydrochloric acid brine (x2), dried and evaporated. The residue was chromatographed on silica to provide the chloride [(36) ; 335mg] as an amorphous solid. $[\alpha]_D^{24} = -27°$ (c = 0.81 in chloroform) $\nu$max ($CHCl_3$) 3350, 1788, 1735cm$^{-1}$. $\delta$ *ppm* ($CDCl_3$) 1.4 (9H, s), 2.05 (3H, s), 2.89 (1H, d, J 9 Hz, exch), 4.57 (1H, s) 4.78 (1H, dd, J 5 Hz and 9 Hz, collapsing to d, J 5 Hz on exch.). 5.67 (1H, d, J 5 Hz), 7.1—7.7 (15H, m), 6.87 (1H, s); (*Found:* C, 67.7; H, 5.8; N, 11.6; Cl, 5.8. $C_{32}H_{32}N_5O_3Cl$ requires C, 67.4; H, 5.7; N, 12.3; Cl 6.2%).

Further elution of the column gave the 8-methylene derivative [(37) ; 475mg] as an amorphous solid $[\alpha]_D^{24} = -22.5°$ (c = 0.96 in chloroform), $\nu$max 3350, 1790, 1735cm$^{-1}$. $\delta$ *ppm* ($CDCl_3$)1.42 (9H, s) 2.55 (1H, d, J 9 Hz), 4.73 (1H, dd, J 4 Hz and 9 Hz, collapsing to d J 4 Hz on exch.), 5.08 b (1H, s), 5.57 b (1H, s), 5.77 (1H, d, J 4Hz), 5.85 b (1H, s), 7.1—7.7 (15H, m), 7.95 (1H, s). $\lambda$max (EtOH) 256nm ($\varepsilon$ 10,300) (*Found:* C, 71.7; H, 6.2; N, 12.7. $C_{32}H_{31}N_5O_3$ requires C, 72.0; H, 5.9; N, 13.1%).

Example 27

(3aR,4S) t-Butyl 4.5-dihydro-5-oxo-8-methyl-4-triphenylmethylamino-3aH-azeto[1,2-a] v-triazolo [3,4-c]pyrimidine-7-carboxylate (38)

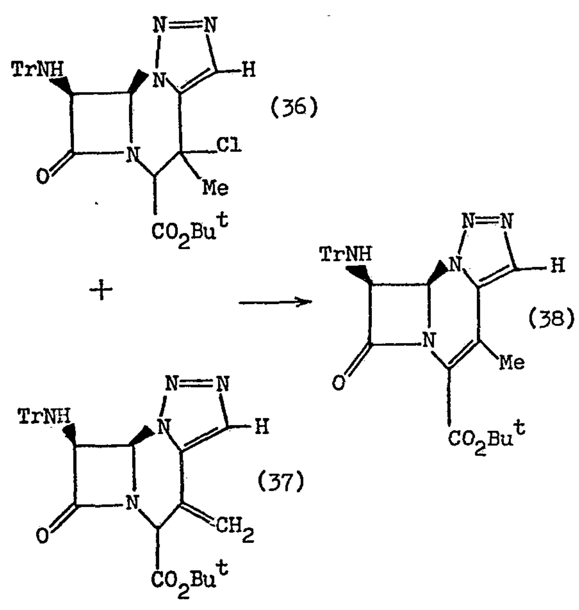

(36) + (37) (38)

The total product from Example 26 [(36) and (37); 810mg] was dissolved in dry methylene chloride (30ml) at −20° and 1,5-diazabicyclo [5,4,0] undecene-5 (DBU) (200mg) added. The reaction mixture was allowed to warm to room temperature and then the volume was reduced to *ca* 10 ml and the mixture filtered through a silica column, to give the product [(38) ; 679mg] as a glass $[\alpha]_D^{23.5} = 2.5°$ (c = 0.74 in chloroform) $\nu$max ($CHCl_3$) 3350, 1800, 1709, 1600cm$^{-1}$. $\delta$ *ppm* ($CDCl_3$)1.5 (9H, s), 2.4 (3H, s), 3.27 (1H, d, J 10 Hz), 4.85—5.15 (2H, m, collapses to s, 5.08 on exch.), 7.1—7.8 (15H, m), 7.83 (1H, s). $\lambda$max (EtOH) 310 nm ($\varepsilon$ 9,100), 231 nm ($\varepsilon$ 18,500). (*Found:* C, 71.5; H, 6.2; N, 12.9. $C_{32}H_{31}N_5O_3$ requires C, 72.0; H, 5.9; N, 13.1%).

Example 28

(3aR,4S) t-Butyl 4,5-dihydro-5-oxo-8-methyl-4-phenoxyacetamido-3aH-azeto [1,2-a] v-triazolo [3,4-c] pyrimidine-7-carboxylate (40)

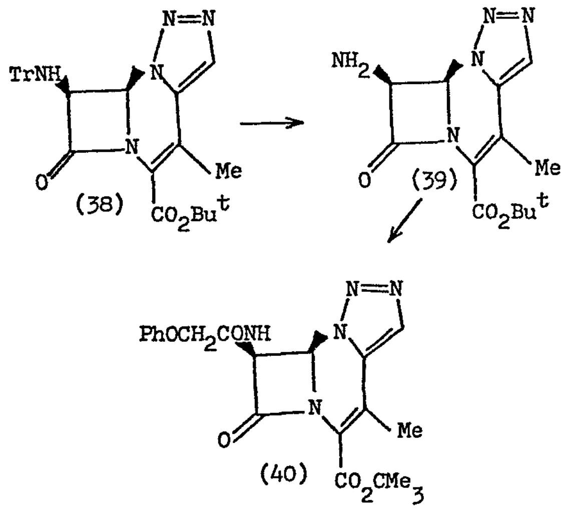

(38) (39) (40)

The lactam [(38) ; 533mg] was dissolved in methylene chloride (10ml) at −20° and toluene-*p*-sulphonic acid (210mg) added in the minimum volume of methanol over 1—2 min. The solution was kept at 6° for 6½hr and the solvents removed. The residue was dissolved in ethyl acetate/aqueous sodium bicarbonate. The organic layer was separated, washed with brine, dried, and evaporated to give (39). The latter was dissolved in methylene chloride (10ml) at −20° and triethylamine (202mg) added, followed by the dropwise addition of phenoxyacetyl chloride (190mg) in methylene chloride (1ml). The solution was washed with brine (x2), dried and evaporated. Chromatography on silica gave the acylamino-derivative [(40) ; 334mg] as a white amorphous solid. $[\alpha]_D^{21.5} = -82.2°$ (c = 1.19 in chloroform). $\nu$max ($CHCl_3$) 3425, 1805, 1700cm$^{-1}$. $\delta$ *ppm* ($CDCl_3$) 1.5 (9H, s), 2.48 (3H, s), 4.45 (2H, s), 5.68—6.03 (2H, m), 6.7—7.7 (6H, m), 7.88 (1H, s), $\lambda$max (EtOH) 310nm ($\varepsilon$ 9,500), 243nm ($\varepsilon$ 6,100) (*Found:* C, 59.3; H, 5.5; N, 16.2. $C_{21}H_{23}N_5O_5$ requires C, 59.3; H, 5.5; N, 16.5%).

Example 29

(3aR,4S) 4,5-Dihydro-5-oxo-8-methyl-4-phenoxyacetamido-3aH azeto[1,2-a] v-triazolo [3,4-c]pyrimidine-7-carboxylate acid (41)

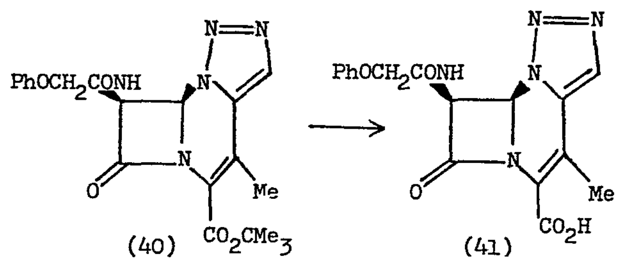

(40) (41)

The ester [(40) ; 216mg] was dissolved in trifluoroacetic acid (8ml) and the solution kept at room temperature for 30 min. The solvent was evaporated off and the residue treated with toluene, and the mixture evaporated; this procedure was repeated. Trituration with ether gave the free acid (41) as a white amorphous solid (174mg). $[\alpha]_D^{20.5} = -61.5°$ (c = 0.93 in DMSO) $\nu$max (KBr) 3370 b, 1795 b, 1690 b cm$^{-1}$. $\delta$ *ppm* ($CDCl_3$) + $D_6$DMSO) 2.46 (3H, s), 4.35 (2H, s), 5.77 (1H, dd, J 3 Hz and 8 Hz, collapses to d J 3 Hz on exch.), 5.97 (1H, d, J 3 Hz), 6.7—7.4 (6H, m, 1H exch.), 7.88 (1H, s), 8.79 (1H, d, J 8 Hz exch.), $\lambda$max (EtOH) 301nm ($\varepsilon$ 7,950), 276nm ($\varepsilon$ 6,440). (*Found:* C, 54.7; H, 4.1; N, 18.4; $C_{17}H_{15}N_5O_5$ requires C, 55.3; H, 4.1; N, 19.0%).

The minimum inhibitory concentrations (MIC) of this compound required to inhibit the growth of various bacteria in nutrient agar are tabulated below:

| Gram-Positive Bacteria | MIC (μg/ml) |
|---|---|
| B. Subtilis | <0.2 |
| Staph. aureus Oxford | 2.5 |
| Staph. aureus Russell* | 5.0 |
| β-Haemolytic Strep. CN 10 | 0.5 |

| Gram-Negative Bacteria | MIC (μ/ml) |
|---|---|
| E. coli JT 4 | 100 |
| Ps. aeruginosa Dalgleish $10^{-2}$ | 100 |
| Klebsiella aerogenes A | 100 |
| Enterobacter cloacae N1 | 100 |
| P. rettgeri | 100 |
| P. mirabilis C977 | >100 |
| P. morganii | >100 |

*β-lactamase producing strain

Example 30

(3aR,4S) 4,5-Dihydro-5-oxo-8-methyl-4-D-mandelylamino-3aH-azeto [1,2-a] v-triazolo [3,4-c] pyrimidine-7-carboxylic acid (42; R = H)

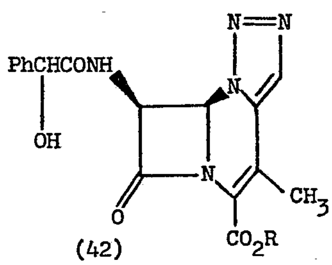

(42)

Reaction of [39; prepared from (38; 269mg)] with D-mandelyl O-carboxyanhydride (100mg) as described in Example 11 afforded the ester [(42); R = $CMe_3$); 166mg]. $[\alpha]_D^{22} = -83.6°$ (C = 1.05, $CHCl_3$). νmax ($CHCl_3$) 3400, 1805, 1705, 1690 (sh) $cm^{-1}$. *δ ppm* ($CDCl_3$)1.55 (9H, s), 2.43 (3H, s), 4.52 b (1H, d, J 3 Hz, exch.), 5.18 (1H, d, J 3Hz, collapses to s on exch.), 5.77—6.0 (2H, m, becomes slightly broadened s at 5.85 on exch.), 7.3 (5H, s), 7.59 (1H, d, J 9 Hz, exch.), 7.65 (1H, s). λmax (EtOH) 311 nm (ε = 8,650). (*Found:* C, 59.3; H, 5.6; N, 16.4. $C_{21}H_{23}N_5O_5$ requires C, 59.3; H, 5.4; N, 16.5%)

Treatment of [(42; R = $CMe_3$); 109mg] with trifluoroacetic acid as described in Example 29 afforded the free acid (42; R = H) as a white amorphous solid (90mg). $[\alpha]_D^{20} = -81.6°$ (C = 0.61 in DMSO). νmax. (KBr) 3360 (b), 1795, 1710, 1675 $cm^{-1}$. *δ ppm* ($D_6$DMSO) 2.44 (3H, s), 4.95 (1H, s), 5.1—6.7 (4H, m, collapses to two d's at 5.83 and 6.0, each 1H, J 4Hz, on exch.), 7.28 b (5H, s), 7.88 (1H, s), 8.72 (1H, d, J 9 Hz, exch.). λmax. (0.3% $NaHCO_3$) 297 (ε = 6,300).

Example 31

(3aR,4S) 4,5-Dihydro-5-oxo-8-methyl-4-(2-thienylacetamido)-3aH-azeto [1,2-a] v-triazolo [3,4-c] pyrimidine-7-carboxylic acid (43; R=H)

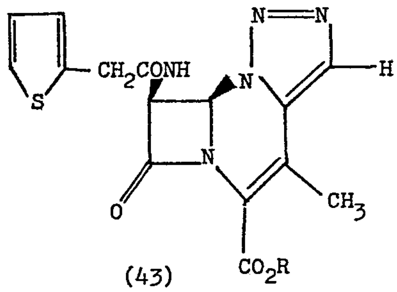

(43)

Acylation of [39; prepared from (38; 268mg)] with 2-thienylacetyl chloride (90mg) as described in Example 28 gave the ester [(43; R = $CMe_3$); 160mg]. $[\alpha]_D^{23} = -87.2°$ (C = 1, $CHCl_3$). $\nu$max. ($CHCl_3$) 3410, 1805, 1708, 1690 (sh) $cm^{-1}$. $\delta$ *ppm* ($CDCl_3$)1.54 (9H, s), 2.44 (3H, s), 3.75 (2H, s), 5.7—5.9 (2H, m), 6.5—6.7 (1H, m), 6.91 (2H, d), 7.2 (1H, q), 7.77 (1H, s). $\lambda$max (EtOH) 310 nm ($\varepsilon$ = 9,070). (*Found:* C, 54.8; H, 5.2; N, 16.7. $C_{19}H_{21}N_5O_4S$ requires: C, 54.9; H, 5.1; N, 16.9%).

Treatment of [(43; R = $CMe_3$); 110mg] with trifluoro-acetic acid as described in Example 29 gave the acid (43; R =H) as a white solid (90mg) $[\alpha]_D^{22°} = -97.6°$ (C = 0.4 in DMSO). $\nu$max. (KBr) 3390, 1790, 1680 $cm^{-1}$. $\delta$ *ppm* ($D_6$DMSO). 2.41 (3H, s), 3.41 b (1H, s), 3.58 (2H, s), 5.8 (1H, dd, J 4Hz and 9Hz), 6.8 (1H, d, J 4Hz), 6.8—7.0 (2H,m), 7.31 (1H, m), 8.16 (1H, s), 8.81 (1H, d, J 9Hz). $\lambda$max. (EtOH) 298 nm ($\varepsilon$ = 8,200).

Example 32

(3aR,4S) 4,5-Dihydro-5-oxo-8-methyl-4-DL ($\alpha$-phenoxycarbonylphenyl-acetamido)-3aH-azeto [1,2-a] v-triazolo [3,4-c] pyrimidine-7-carboxylic acid (44; R = H)

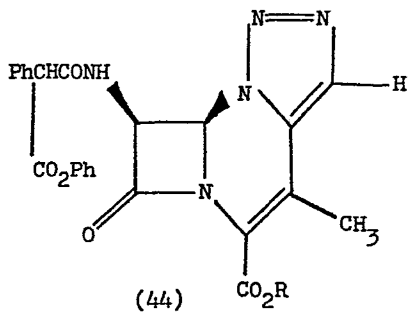

(44)

Reaction of [39 ; prepared from (38; 268mg)] with freshly prepared DL-$\alpha$-phenoxycarbonylphenylacetyl chloride (150mg) as described in Example 29 afforded the amorphous ester [(44; R = $CMe_3$); 223 mg]. $[\alpha]_D^{20} = -39.1°$ (c = 1.03, $CHCl_3$) $\nu$max. 3350, 1805, 1740, 1710, 1690 (sh) $cm^{-1}$. $\delta$ *ppm* ($CDCl_3$) 1.55 (9H, s), 2.45 (3H, s), 4.69 and 4.77 (together 1H, s), 5.7—5.9 (2H,m), 6.9—7.7 (11H, m), 7.78 and 7.81 (together 1H, s). $\lambda$max (EtOH) 330 nm ($\varepsilon$ = 6,580). (*Found:* C, 63.6; H, 5.3; N, 13.3. $C_{28}H_{27}N_5O_6$ requires: C, 63.5; H, 5.1; N, 13.2%).

Trifluoroacetic acid treatment of [(44; R = $CMe_3$); 113 mg] as described in Example 29 gave the free acid (44; R = H) as a white solid (95mg). $[\alpha]_D^{22} = -53.4°$ (C = 0.86 in DMSO). $\nu$max. (KBr) 3325, 1797, 1770 (sh) 1690 $cm^{-1}$. $\delta$ *ppm* ($D_6$DMSO). 2.43 (3H, s), 3.3 b (1H, s, exch), 4.88 and 4.92 (together 1H, s), 5.7—6.0 (1H, m), 6.12 (1H, t, overlapping doublets of $\beta$-lactam of each epimer), 7.0—7.5 (10H, m), 8.12 and 8.18 (together 1H, s), 9.05—9.3 (1H,m). $\lambda$max. (EtOH) 301 nm ($\varepsilon$ = 7,300).

Example 33

(3aR,4S)-4,5-Dihydro-5-oxo-8-methyl-4-D-$\alpha$-phenylglycylamino-3aH-azeto [1,2-a] v-triazolo [3,4-c] pyrimidine-7-carboxylic acid trifluoroacetic acid salt (46)

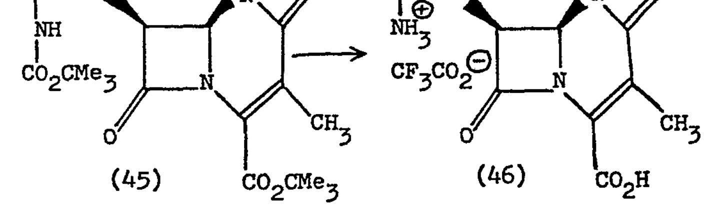

(45) (46)

Acylation of [39; prepared from (38; 268mg)] as described in Example 14 provided the ester (45; 260mg). $[\alpha]_D^{20°} = -78.8°$ (C = 0.95, $CHCl_3$). νmax. ($CHCl_3$) 3430, 1807, 1710, 1690 (sh) $cm^{-1}$. δ *ppm* ($CDCl_3$) 1.37 (9H, s), 1.53 (9H, s), 2.45 (3H, s), 5.25 (1H, d, J 7 Hz), 5.49 (1H, d, J 7 Hz), 5.65 (1H, dd, J 4 Hz and 9 Hz, collapses to d, J 4 Hz on exch.), 5.82 (1H, d, J 4 Hz), 7.29 (6H, m, 1 H exch.), 7.77 (1H, s). λmax. (EtOH) 310nm (ε = 8,940). (*Found:* C, 59.3; H, 6.1; N, 16.1. $C_{26}H_{32}N_6O_6$ requires: C, 59.5; H, 6.1; N, 16.0%).

Treatment of (45; 156mg) with trifluoroacetic acid as described in Example 29 gave the free acid (46) as an off-white amorphous solid (133 mg). $[\alpha]_D^{20} = -65.7°$ (C = 0.99 in DMSO). νmax. (KBr) 3400, 3000b, 1795, 1670b, $cm^{-1}$. δ *ppm* ($CDCl_3$ + $D_6DMSO$) 2.44 (3H, s), 4.93 (1H, s), 3.0—5.5 (4H, very broad s, exch.) 5.7—6.2 (2H, m, collapses to two d's at 5.81 and 6.02, J 4 Hz on exch.), 7.4 (5H, s), 8.01 (1H, s), 9.24 (1H, d, J 8 Hz, exch.) λmax. (EtOH) 312 nm (ε = 4,000).

Example 34

(3aR,4S)-4,5-Dihydro-5-oxo-8-methyl-4-(2-tetrazolylacetamido)-3aH-azeto [1,2-a] v-triazolo [3,4-c] pyrimidine-7-carboxylic acid (47; R = H)

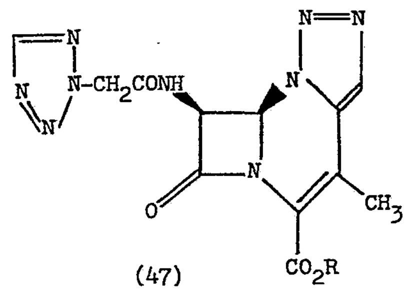

(47)

Acylation of [39 ; prepared from (38; 269mg)], with freshly prepared 2-tetrazolylacetyl chloride (145 mg) as described in Example 28 gave the ester [(47; R = $CMe_3$); 165 mg]. $[\alpha]_D^{23} = -71.4°$ (c = 0.6 in $CHCl_3$). νmax ($CHCl_3$) 3225 b, 1805, 1710, 1685 sh $cm^{-1}$. δ *ppm* ($CDCl_3$) 1.57 (9H, s), 2.43 (3H, s), 5.6 (2H, s), 5.8—6.2 (2H, m), 7.66 (1H, s), 8.29 (1H, d, J 8Hz), 8.55 (1H, s). λmax (EtOH) 308 nm (ε = 9,370). (*Found:* C, 48.2; H, 5.1. $C_{16}H_{19}N_9O_4$ requires C, 47.9; H, 4.7%).

Trifluoroacetic acid treatment of [(47; R = $CMe_3$); 145 mg] as described in Example 29 gave the free acid (47; R = H) as a white solid (118 mg). $[\alpha]_D^{21.5} = 65.8°$ (C 1.4 in DMSO). νmax. (KBr) 3320, 1793, 1705 $cm^{-1}$. δ*ppm* ($D_6DMSO$) 2.40 (3H, s), 5.42 (2H, s), 5.90 (2H, dd, J 8Hz and 3½Hz), 6.12 (1H, d, J 3½Hz), 6.5—8.0b (1H, s, exch.), 8.17 (1H, s) 8.92 (1H, s), 9.26 (1H, d, J 8 Hz). λmax. (0.3% $NaHCO_3$) 294 nm (ε = 7,050).

Example 35

(3aR,4S)-4,5-Dihydro-5-oxo-8-methyl-4-ethylthioacetamido-3aH-azeto [1,2-a] v-triazolo [3,4-c] pyrimidine-7-carboxylic acid (48; R=H).

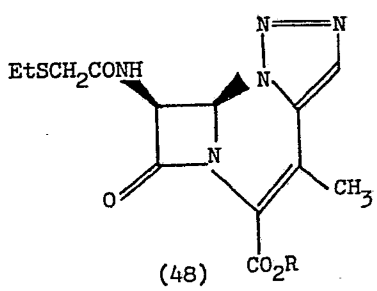

(48)

Acylation of [39; prepared from (38; 268 mg)] with freshly prepared ethylthioacetyl chloride (77 mg) as described in Example 28 afforded the ester [(48; R = $CMe_3$); 80 mg]. $[\alpha]_D^{23} = -103.5°C$ (C = 1.02 in $CHCl_3$). νmax. ($CHCl_3$) 3360, 1805, 1710, 1686 $cm^{-1}$. δ *ppm* ($CDCl_3$) 1.23 (3H, t, J 7½Hz), 1.57 (9H, s), 2.48 (3H, s), 2.59 (2H, q, J 7½Hz), 3.2 (2H, s), 5.8—6.0 (2H, m, becomes sharp s, 5.9 on irradiation at 7.52), 7.4—7.65 (1H, m), 7.85 (1H, s). λmax. (EtOH) 311nm (ε = 8,950). (*Found:* C, 51.6; H, 6.0; N, 16.5. $C_{17}H_{22}N_5O_4S$ requires: C, 51.9; H, 5.9; N, 17.8%).

Trifluoroacetic acid treatment of [(48; R = $CMe_3$); 75 mg] as described in Example 29 gave the free acid (48; R = H). as a white amorphous solid (45mg). $[\alpha]_D^{25.5} = -100.0°$ (C 1.0 in DMSO). νmax. (KBr) 3130, 1800 b, 1698, 1670 $cm^{-1}$. δ *ppm* 1.13 (3H, t, J 7½Hz), 2.43 (3H, s), methylene of S—$CH_2$—$CH_3$ is obscured by solvent signals, 3.04 (2H, s), 2.7—3.7 (very broad s, 1H, exch.), 5.78 (1H, dd, J 9Hz and 3½Hz), 8.15 (1H, s), 8.76 (1H, d, J 9Hz). λmax. (0.3% $NaHCO_3$) 296 nm (ε = 9.120).

Example 36

(3aRF,4S)-4,5-Dihydro-5-oxo-8-methyl-4-cyanoacetamido-3aH-azeto [1,2-a] v-triazolo [3,4-c] pyrimidine-7-carboxylic acid (49; R=H)

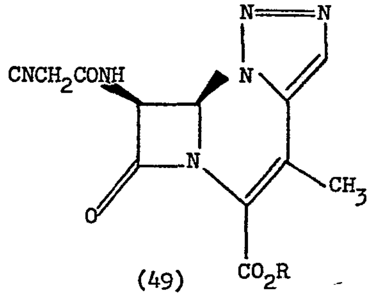

(49)

The free base [39; prepared from 38; 268 mg)] was dissolved in dry methylene chloride (5 ml) at 0°C. Dicyclohexyl-carbodiimide (113 mg) in dry methylene chloride (1 ml) was added, followed by the dropwise addition of cyanoacetic acid (46 mg) in dry DMF (1 ml). After 2 h the mixture was diluted with methylene chloride and then washed successively with dilute hydrochloric acid, dilute aqueous sodium bicarbonate and brine. The organic layer was dried and evaporated. Chromatography of the residue on silica afforded the ester [(49; R = CMe$_3$) 100 mg]. $[\alpha]_D^{22} = -93.62°$ (C 0.82 in CHCl$_3$). νmax. (CHCl$_3$) 1806, 1708, 1690 sh cm$^{-1}$. δ *ppm* (CDCl$_3$)1.56 (9H, s), 2.44 (3H, s), 3.48 (2H, s), 5.89 (2H, m, becomes sharp s on irradiation at 7.95), 7.72 (1H, s), 7.8—8.04 (1H, m). λmax. (EtOH) 306 nm (ε = 10,050). (*Found:* C, 53.7; H, 5.0; N, 22.4. $C_{16}H_{18}N_6O_4$ requires: C, 53.6; H, 5.0; N, 23.5%).

Trifluoroacetic acid treatment of [(49; R=CMe$_3$); 60mg] as described in Example 29 gave the acid (49 ; R=H) as an amorphous solid (40mg). $[\alpha]_D^{25.5} = -96.4°$ (C 1.04 in DMSO). νmax. (KBr) 3350 b, 1800, 1680 b cm$^{-1}$. δ *ppm* (D$_6$DMSO) 2.42 (3H, s), 3.58 (2H, s), 3.0—5.0 b (1H, s, exch.) 5.82 (1H, dd, J 8Hz and 3½Hz, becomes d, J 3½Hz on exch.), 8.18 (1H, s), 9.04 (1H, d, J 8 Hz, exch.), λmax. (CH$_3$CN) 305 nm (ε 8,700).

Example 37

(3aR, 4S)-4,5-Dihydro-5-oxo-8-methyl-4-[(2-methoxyimino-2-fur-2′-yl) acetamido]-3aH-azeto (1,2-a] v-triazolo [3,4-c] pyrimidine-7-carboxylic acid (50; R = H)

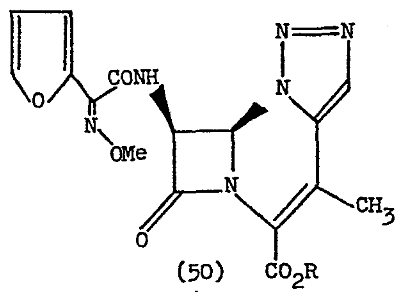

(50)

Treatment of [39; prepared from (38; 268 mg)] with dicyclohexylcarbodiimide/2-(fur-2-yl)-2-(syn)methoxyiminoacetic acid as described in Example 36 gave the ester [(50; R = CMe$_3$) 100 mg]. $[\alpha]_D^{25.5} = -65°$ (C 1.04 in CHCl$_3$). νmax. (CHCl$_3$) 3400, 1806, 1710, 1690 sh, 1651 cm$^{-1}$. δ *ppm* (CDCl$_3$) 1.57 (9H, s), 2.5 (3H, s), 4.02 (3H, s), 5.9—6.10 (2H, m, becomes s, 6.0 on exch.), 6.43 (1H, dd, J 3Hz and *ca.* 1½Hz), 6.91 (1H, d, J 3Hz), 7.38 b (1H, s, exch.), 7.45 (1H, d, J *ca.* 1½Hz), 7.86 (1H, s). λmax. (EtOH) 285 nm (ε = 17,400).

Deprotection of [(50; R = CMe$_3$); 90 mg] with trifluoroacetic acid as described in Example 29 afforded the acid (50; R = H) as a white powder (60 mg). $[\alpha]_D^{21.5} = -12.3°$ (C = 1.0 in DMSO). νmax. (KBr) 3350, 1800, 1678cm$^{-1}$. δ *ppm* (D$_6$DMSO) 2.4 (3H, s), 3.32 b (1H, s, exch.), 3.84 (3H,s), 5.95 (1H, dd, J 8 Hz and 3½Hz, becomes d, J 3½Hz on irradiation at 9.48), 6.2 (1H, d, J 3½Hz), 6.6 (1H, m), 6.79 (1H, d, J 3 Hz),7.77 (1H, slightly broadened s), 8.18 (1H, s), 9.43 (1H, d, J 8Hz). λmax. (1% NaHCO$_3$) 288 nm (ε = 19,800).

Example 38

(3aR,4S)-4,5-Dihydro-5-oxo-8-methyl-4-trifluoroacetamido-3aH-azeto [1,2-a] v-triazolo [3,4-c] pyrimidine-7-carboxylic acid (51; R = H).

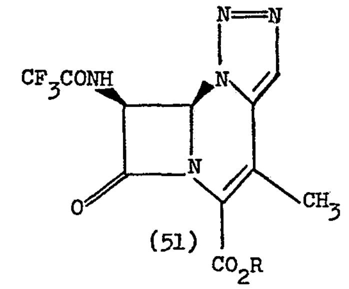

(51)

The free base [39; prepared from (38; 269 mg)] was dissolved in dry methylene chloride (5 ml) and triethylamine (76 mg) added, followed by the dropwise addition of trifluoroacetic anhydride (158 mg) in dry methylene chloride (5 ml). The mixture was washed successively with dilute hydrochloric acid, dilute aqueous sodium hydrogen carbonate and brine, dried and evaporated. Chromatography on silica afforded the ester (51; R = $CMe_3$) as an amorphous solid (117 mg). $[\alpha]_D^{22} = -106.1°$ (C 1.0 in $CHCl_3$) νmax. ($CHCl_3$) 1806, 1710 b $cm^{-1}$. δ($CDCl_3$) 1.57 (9H, s), 2.57 (3H, s), 3.9—4.14 (2H, m), 7.75 (1H, s), 8.06 (1H, d, J 9 Hz). λmax. (EtOH) 307 nm (ε = 10,200).

Deprotection of [(51; R = $CMe_3$); 75 mg] with trifluoroacetic acid as described in Example 29 provided the acid (51 ; R = H) as a white solid (55 mg). $[\alpha]_D^{25.5} = -128.2°$ (c 1.0 in DMSO).νmax. (KBr) 3290, 1800, 1718 $cm^{-1}$. *δ ppm* ($D_6DMSO$) 2.42 (3H, s), 2.7—3.7 (1H, m, exch.), 5.94 (1H, dd, J 8Hz and 3½Hz, becomes d, J 3½Hz on exch.), 4.18 (1H, d, J 3½Hz), 8.18 (1H, s), 10.2 (1H, d, J 8 Hz exch.). λmax ($CH_3CN$) 3.4 nm (ε = 10,350).

Exampie 39

(3aR,4S) t-Butyl 4,5-dihydro-5-oxo-8-bromomethyl-4-triphenylmethylamino 3aH-azeto [1,2-a] v-triazolo [3,4-c] pyrimidine-7-carboxylate (52)

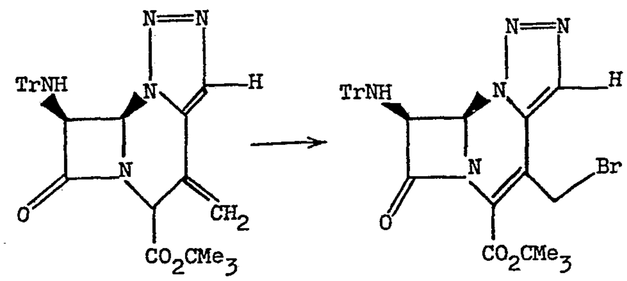

(37) (52)

The lactam (37; 618 mg) was dissolved in dry tetrahydrofuran (40 ml) containing dibromodiethylmalonate (400 mg). The solution, under argon, was cooled to −76° and 1.5-diazabicyclo [5.4.0] undec-5-ene (193 mg) in dry tetrahydrofuran (10 ml) added dropwise over 10 minutes. The solution was poured into ethyl acetate—very dilute hydrochloric acid and the organic layer separated, washed with brine, dried and evaporated. Chromatography on silica afforded the bromo-derivative (52) as a white crystalline solid (486mg). m.p. 162—163° dec. (ether trituration) $[\alpha]_D^{20} = -27.6°$ (C=1. $CHCl_3$). νmax. ($CHCl_3$) 3350, 1805, 1712 $cm^{-1}$. *δ ppm* ($CDCl_3$) 1.38 (9H, s), 3.10 (1H, d, J 9Hz exch.), 4.40 and 4.68 (2H, ABq, J 10Hz), 4.78—5.0 (2H, m, collapses to s at 4.92 on exch.), 6.9—7.5 (15H, m), λmax. (EtOH) 320 nm (ε = 8,640). (*Found:* C, 63.1; H, 5.2; N, 11.4, Br, 13.3. $C_{32}H_{30}N_5O_3Br$ requires: C, 62.7; H, 4.9; N, 11.4; Br, 13.1%).

Example 40

(3aR,4S) t-Butyl, 4,5-dihydro-5-oxo-8-acetoxymethyl-4-triphenylmethylamino-3aH-azeto [1,2-a] v-triazolo [3,4-c] pyrimidine-7-carboxylate (54)

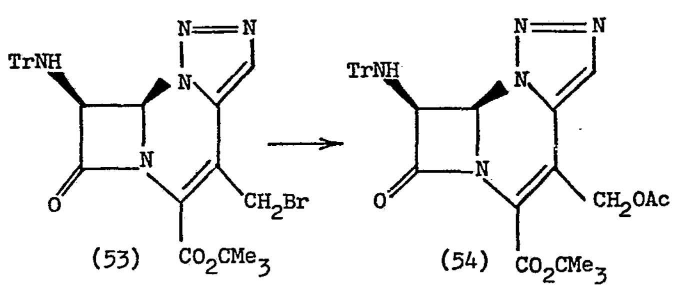

(53) (54)

The lactam (53; 314mg) was dissolved in chloroform (10 ml) (dried by passage through basic alumina) and tetraethylammonium acetate (200 mg) added. The mixture was stirred in the dark for 21 h and then washed with dilute hydrochloric acid, brine, dried and evaporated. Chromatography on silica afforded (54) as an amorphous solid (255mg) $[\alpha]_D^{21} = -19.3°$ (C = 1 in $CHCl_3$). νmax ($CHCl_3$) 3350, 1802, 1740, 1715 $cm^{-1}$. *δ ppm* ($CDCl_3$) 1.5 (9H, s), 2.03 (3H, s), 3.28 (1H, d, J 10Hz exch.), 4.95—5.15 (2H, m, collapses to s at 5.08 on exch.), 5.09 and 5.58 (2H, ABq, J 14 Hz), 7.0—7.75 (15H, m), 7.89 (1H, s). λmax (EtOH) 311 nm (ε = 9,350). (*Found:* C, 69.0; H, 5.7; N, 11.6. $C_{34}H_{33}N_5O_5$ requires C, 69.0; H, 5.6; N, 11.8%).

Example 41

(3aR,4S) t-Butyl 4.5-dihydro-5-oxo-8-acetoxymethyl-4-(2-thienylacetamido)-3aH-azeto [1,2-a] v-triazolo [3,4-c] pyrimidine-7-carboxylate (56)

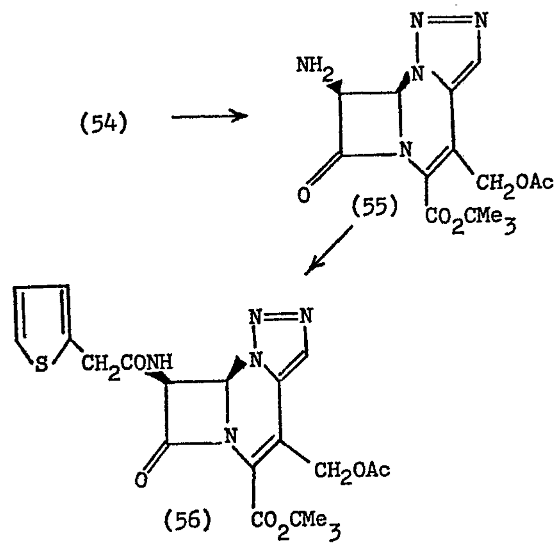

(54)

(55)

(56)

The lactam (54; 261 mg) was dissolved in dry methylene chloride (8ml) and the solution cooled to −20°. Toluene-p-sulphonic acid (92mg) was then added in the minimum volume of methanol and the solution kept at 4°C for 18 h. The solvents were removed and the residue dissolved in ethyl acetate—sodium hydrogen carbonate. The organic layer was separated, washed with brine, dried, and evaporated to give (55). The latter was acylated with 2-thienylacetyl chloride (70 mg) as described in Example 28, to provide (56; 58mg). $[\alpha]_D^{21.5} = -136.7°$ (C = 0.39 in $CHCl_3$). νmax ($CHCl_3$) 3415, 1810, 1740, 1713, 1689 $cm^{-1}$. *δ ppm* ($CDCl_3$) 1.58 (9H, s), 2.08 (3H, s), 3.77 (2H, s), 5.17 and 5.7 (2H, ABq, J 14Hz), 5.67—6.0 (2H, m), 6.82—7.41 (3H, m), 7.9 (1H, s). λmax. (EtOH) 315 nm (ε = 9,370).

Example 42

(3aR,4S) 4,5-Dihydro-5-oxo-8-acetoxymethyl-4-(2-thienylacetamido)-3aH-azeto [1,2-a] v-triazolo [3,4-c] pyrimidine-7-carboxylic acid (57)

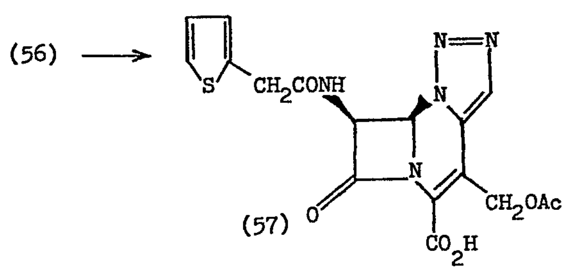

(56)

(57)

The ester (56; 35 mg) was treated with trifluoroacetic acid (2ml) as described in Example 29 to provide the free acid (57) as a tan coloured solid (28mg). $[\alpha]_D^{21.5} = -115.1°$ (C = 0.23 in DMSO). νmax. (KBr) 3350, 1805, 1725, 1675 $cm^{-1}$. *δ ppm* ($D_6$DMSO) 2.03 (3H, s) 3.58 (2H, s), 3.75—5.25 b (1H, s, exch.), 5.16 and 5.42 (2H, ABq, J 13 Hz), 5.83 (1H, dd, J 8 Hz and 3½Hz), 6.13 (1H, d, J 3½ Hz), 6.75—7.40 (3H, m), 8.1 (1H, s), 8.85 (1H, d, J 8Hz), λmax (0.3% $NaHCO_3$) 300nm (ε = 6,630).

Example 43

(3aR,4S) t-Butyl 4,5-dihydro-5-oxo-8-[5-methyl (1,3,4-thiadiazol-2-yl) thiomethyl]-4-triphenylmethylamino-3aH-azeto [1,2-a] v-triazolo [3,4-c] pyrimidine-7-carboxylate (58)

(53)

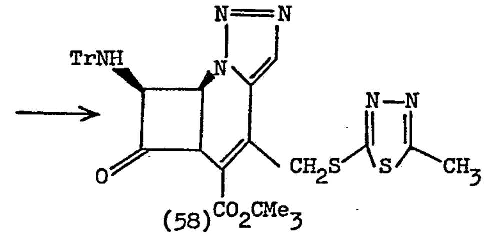

(58)

The lactam (53; 306mg) was dissolved in dry methylene chloride (10ml) and 5-methyl-2-mercapto-1,3,5-thiadiazole (80mg) added. The solution was cooled to −20° and triethylamin (60mg) in methylene chloride (2ml) added. The reaction was allowed to warm to room temperature and was then washed with dilute hydrochloric acid, brine, dried and evaporated. Chromatography on silica afforded the ester (58) as an amorphous solid (288mg). $[\alpha]_D^{20} = -60.1$ (C = 0.89 in $CHCl_3$). $\nu$max. ($CHCl_3$) 3350, 1802, 1710 $cm^{-1}$. $\delta$ *ppm* ($CDCl_3$) 1.5 (9H, s), 2.65 (3H, s), 3.23 (1H, d, exch.), 4.58 and 4.98 (2H, ABq, J 13Hz), 4.9—5.1 (2H, m, collapses to s at 5.0 on exch.), 7.0—7.7 (15H, m), 8.04 (1H, s). $\lambda$max (EtOH) 316 nm ($\varepsilon = 8{,}900$). (*Found:* C, 63.0; H, 5.2; N, 14.3. $C_{35}H_{33}N_7O_3S_3$ requires: C, 63.3; H, 5.0; N, 14.8%).

## Example 44

(3aR,4S) t-Butyl 4,5-dihydro-5-oxo-8-[5-methyl (1,3,4-thiadiazol-2-yl) thiomethyl]-4-(2-thienylacetamido)-3aH-azeto [1,2-a] v-triazolo [3,4-c] pyrimidine-7-carboxylate (59)

(58)

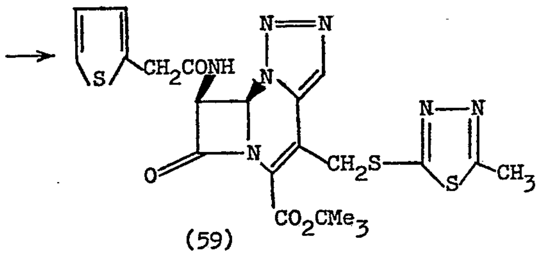

(59)

Detritylation and acylation of (58; 221 mg) as described in Example 41 gave (59; 115mg). $[\alpha]_D^{22} = -130.8°$ (C 1.0 in $CHCl_3$). $\nu$max ($CHCl_3$) 3410, 1805, 1700 b $cm^{-1}$. $\delta$ *ppm* ($CDCl_3$) 1.56 (9H, s) 2.71 (3H, s), 3.76 (2H, s), 4.62 and 5.09 (2H, ABq, J 13Hz), 5.72—5.89 (2H, m), 6.55 (1H, d, J 7½Hz), 6.92—7.26 (3H, m), 8.05 (1H, s). $\lambda$max. 321nm ($\varepsilon = 9{,}360$). (*Found:* C, 48.4; H, 4.0; N, 17.2. $C_{22}H_{23}N_7O_4S_3$ requires: C, 48.4; H, 4.2; N, 18.0%).

## Example 45

(3aR,4S) 4,5-Dihydro-5-oxo-8-[5-methyl (1,3,4-thiadiazol-2-yl) thiomethyl]-4-(2-thienylacetamido)-3aH-azeto [1,2-a]-v-triazolo [3,4-c] pyrimidine-7-carboxylic acid (60)

(59)

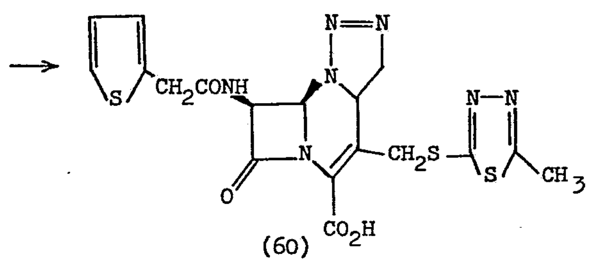

(60)

The ester (59; 69mg) was treated with trifluoroacetic acid (2ml) as described in Example 29 to give the acid (60) as a pale green-grey solid (62 mg). $[\alpha]_D^{21.5} = -157.6°$ (C = 0.79 in DMSO). $\nu$max (KBr) 3370, 1800, 1710 sh, 1680 $cm^{-1}$. $\delta$ *ppm* ($D_6$DMSO) 2.67 (3H, s) 3.48 and 3.68 (2H, ABq, J 16Hz), 3.20—3.7 b (1H, s, exch.), 4.57 and 5.03 (2H, ABq, J 14 Hz), 5.74 (1H, dd, J 8Hz and 3½Hz, becomes d, J 3½Hz on exch.), 6.06 (1H, d, J 3½Hz), 6.8—7.4 (3H, m), 8.18 (1H, s), 8.88 (1H, d, J 8Hz, slowly exch.). $\lambda$max (0.3% $NaHCO_3$) 300 nm ($\varepsilon = 7{,}500$).

The antibacterial activity of the compounds described in Examples 30—38, 42 and 45 are set out in Tables 1 and 2.

TABLE 1

MIC (μg/ml)*

| Organism | Compounds (R = H) | | | | | | |
|---|---|---|---|---|---|---|---|
| | 42 | 43 | 44 | 46 | 47 | 57 | 60 |
| E. coli JT 4 | >100 | >100 | >100 | >100 | >100 | >100 | >100 |
| K. aerogenes A | >100 | 50 | 50 | >100 | >100 | >100 | >100 |
| E. cloacae N1 | >100 | >100 | >100 | >100 | >100 | >100 | >100 |
| P. rettgeri | >100 | >100 | 50 | >100 | >100 | >100 | >100 |
| P. mirabilis C 977 | >100 | 10 | 50 | >100 | >100 | >100 | >100 |
| P. morganii | 100 | >100 | >100 | >100 | >100 | >100 | >100 |
| B. subtilis | 1 | <0.2 | 25 | 100 | 0.5 | 100 | 50 |
| S. aureus Oxford | 25 | 1 | 50 | >100 | 2.5 | >100 | >100 |
| S. aureus Russell | >100 | 2.5 | 100 | >100 | 5.0 | >100 | >100 |
| S. aureus 1517 | >100 | 2.5 | >100 | >100 | >100 | >100 | >100 |
| S. faecalis 1 | >100 | 2.5 | >100 | >100 | 50 | >100 | >100 |
| S. pyogenes CN 10 | – | <0.2 | – | – | 1.0 | >100 | >100 |

* Serial dilution in nutrient agar containing 5% horse blood, inoculum 0,001 ml undiluted broth culture.

TABLE 2

MIC (μg/ml)*

| Compounds | Compounds (R = H) | | | | | | |
|---|---|---|---|---|---|---|---|
| | 43 | 44 | 48 | 49 | 50 | 51 | 60 |
| E. coli 0111 | 10 | 10 | 50 | 100 | 100 | 100 | >100 |
| K. aerogenes A | 2.5 | 10 | 50 | 100 | 100 | >100 | >100 |
| E. cloacae N1 | >100 | 50 | >100 | >100 | >100 | >100 | >100 |
| P. rettgeri WM 16 | >100 | 10 | >100 | >100 | >100 | >100 | >100 |
| P. mirabilis C 977 | 2.5 | 10 | 10 | 50 | 50 | >100 | >100 |
| P. morganii 1580 | 2.5 | 10 | 25 | 100 | 100 | >100 | >100 |
| B. subtilis | ≤0.2 | 25 | 0.2 | 2.5 | 5.0 | 5.0 | 5.0 |
| S. aureus Oxford | 2.5 | 25 | –[c] | 5.0 | –[c] | >100 | –[c] |
| S. aureus Russell | 1.0 | 25 | 5.0 | 25 | 25 | >100 | 25 |
| S. aureus 1517 | 5.0 | 100 | 25 | 100 | 100 | >100 | 100 |
| S. faecalis 1 | 5.0 | >100 | 25 | 25 | >100 | 100 | 25 |
| S. pneumoniae CN 33 | ≤0.2 | 10 | 1.0 | 5.0 | 10 | 50 | 0.5 |
| S. pyogenes CN 10 | ≤0.2 | 10 | 0.5 | 2.5 | 1.0 | 25 | 2.5 |

* Serial dilution in DST agar containing 10% horse blood, inoculum 0.001 ml broth culture diluted $10^{-2}$ for Gram-positive and $10^{-4}$ for Gram-negative organisms.

–[c] Culture contaminated.

## Claims

1. A compound of the formula (I):

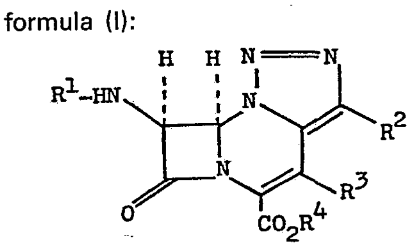

(I)

wherein $R^1$ is a hydrogen atom, a trityl group or an acyl group as found in known antibacterially active penicillins or cephalosporins; $R^2$ is a hydrogen atom, a $C_{1-4}$ alkyl group, phenyl, pyridyl or phenyl substituted by a fluorine, chlorine or bromine atom or a nitro, $C_{1-4}$ alkyl, $C_{1-4}$ alkoxyl, carboxylic acid group or a $C_{1-4}$ alkyl or benzyl ester thereof; $R^4$ is a group such that $CO_2R^4$ is a carboxylic acid group or a salt or readily removable ester thereof. $R^3$ is a hydrogen atom, a $C_{1-4}$ alkyl group optionally substituted by a $CO_2R^4$, $OR^5$ or $SR^7$ group wherein $R^4$ is as defined hereinbefore, $R^5$ is a $C_{1-4}$ alkyl or $C_{1-4}$ acyl group, and $R^7$ is a 5-membered heteroaromatic ring containing up to 4 heteroatoms any of which may be N, and one of which may be O or S and which may be optionally substituted by a $C_{1-4}$ alkyl group optionally substituted by a $CO_2R^4$ or $OR^5$ group.

2. A compound as claimed in Claim 1 of the formula II:

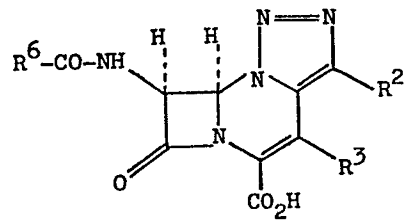

(II)

or a salt thereof wherein $R^2$ and $R^3$ are as defined in relation to formula (I) in claim 1 and $R^6$ is a group such that $R^6$—CO—NH— is an acylamino group as found in antibacterially active penicillins or cephalosporins.

3. A compound as claimed in claim 2 wherein $R^3$ is a methyl group.

4. A compound as claimed in claim 2 of the formula (III):

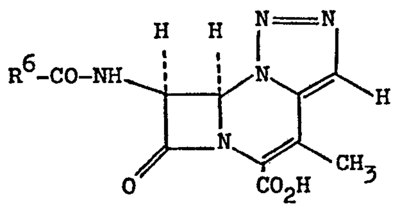

(III)

or a salt thereof wherein $R^6$ is as defined in relation to formula (II) in claim 2.

5. A compound as claimed in claim 5 wherein $R^6$.CO is of the sub-formulae (a)—(d):

$$A_1 - (CH_2)_n - \underset{\displaystyle X}{\overset{|}{CH}} - (CH_2)_m - CO \qquad (a)$$

$$A_2 - CO \qquad (b)$$

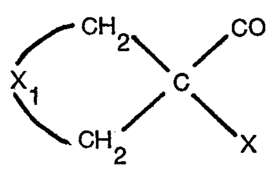

(c)

$$A_2 - X_2 - (CH_2)_n - CO \qquad (d)$$

wherein n is 0, 1 or 2; m is 0, 1 or 2; $A_1$ is $C_1$—$C_6$ alkyl, $C_3$—$C_6$ cycloalkyl, cyclohexenyl, cyclohexadienyl, phenyl, hydroxy-phenyl, thienyl or pyridyl group; X is a hydrogen or halogen atom, a carboxylic acid, carboxylic ester, azido, tetrazolyl, hydroxy, acyloxy, amino, ureido, guanidino or acylureido group; $A_2$ is a phenyl, a 2,6-dimethoxyphenyl, 2-alkoxy-1-naphthyl, 3-arylisoxazolyl or 3-aryl-5-methylisoxazolyl group; $X_1$ is a $CH_2OCH_2$, $CH_2SCH_2$ or $(CH_2)_n$ group; and $X_2$ is an oxygen or sulphur atom.

6. A compound as claimed in claim 5 wherein $R^6$ is a group of the sub-formulae (e) or (f):—

$$R^7—\underset{\displaystyle R^8}{\underset{|}{CH}}— \qquad (e)$$

$$R^9—\underset{\displaystyle R^{10}}{\underset{|}{CH}}— \qquad (f)$$

wherein $R^7$ is a phenyl, thienyl or phenoxy group; $R^8$ is a hydrogen atom or methyl group; $R^9$ is a phenyl, p-hydroxyphenyl, thienyl or cyclohexadienyl group; and $R^{10}$ is a hydroxyl, amino or carboxylic acid group or a lower alkyl or phenyl, tolyl or indanyl ester thereof.

7. A compound as claimed in claim 6 wherein $R^6CO$ is a D-phenylglycyl, D-p-hydroxyphenylglycyl, D-mandelyl, malonyl, benzoyl, 2-thienylacetyl, 3-thienylacetyl, 2-thienyl-$\alpha$-carboxyacetyl, 3-thienyl-$\alpha$-carboxyacetyl or phenoxyacetyl group.

8. A compound as claimed in claim 1 wherein $R^1$ is a hydrogen atom.

9. A pharmaceutical composition which comprises a compound as claimed in claim 2 and a pharmaceutically acceptable carrier therefor.

10. A process for the preparation of a compound as claimed in claim 2 which process comprises the acylation of a compound of the formula (V):

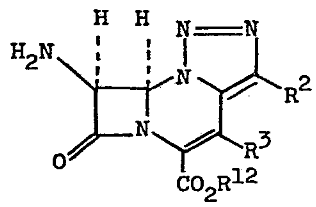
(V)

wherein $R^2$ and $R^3$ are as defined in claim 2 and $R^{12}$ is an ester readily convertible to a carboxylic acid or a salt thereof by hydrogenolysis or hydrolysis; with a reactive acylating derivative of a carboxylic acid of the formula (VI):

$$R^6—CO_2H \quad \text{(VI)}$$

wherein $R^6$ is as defined in relation to formula (II) in claim 2 and in which any acylatable group is optionally protected and thereafter performing one or more of the following reactions:

(a) removing any protecting group from the group $R^6$

(b) hydrolysing or hydrogenolysing the ester group $CO_2R^{12}$ to yield a free or salted carboxylic group.

11. A process for the preparation of a compound of the formula (V) in claim 10 which comprises detritylation of a corresponding compound of the formula (IV):

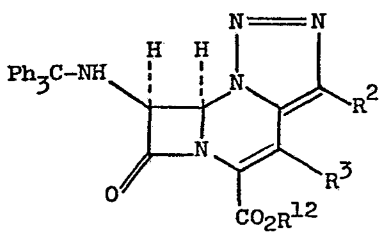
(IV)

wherein $R^2$, $R^3$ and $R^{12}$ are as defined in claim 10.

**Revendications**

1. Composé de formule (I):

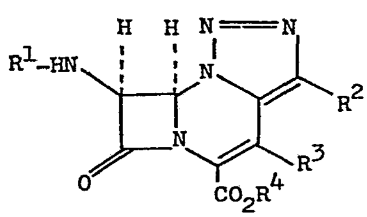
(I)

dans laquelle $R^1$ est un atome d'hydrogène, un groupe trityle ou un groupe acyle tel qu'on le trouve dans les pénicillines ou les céphalosporines à activité antibactérienne; $R^2$ est un atome d'hydrogène, un groupe alcoyle en $C_{1-4}$, phényle, pyridyle ou phényle substitué par un atome de fluor, de chlore ou de brome ou par un groupe nitro, alcoyle en $C_{1-4}$, alcoxy en $C_{1-4}$, acide carboxylique ou ester d'alcoyle en $C_{1-4}$ ou de benzyle de celui-ci ; $R^4$ est un groupe tel que $CO_2R^4$ soit un groupe acide carboxylique ou un sel ou un ester aisément éliminable de celui-ci ; $R^3$ est un atome d'hydrogène, un groupe alcoyle en $C_{1-4}$ éventuellement substitué par un groupe $CO_2R^4$ $OR^5$ ou $SR^7$, où $R^4$ a la même signification que précédemment, $R^5$ est un groupe alcoyle en $C_{1-4}$ ou acyle en $C_{1-4}$, et $R^7$ est un cycle hétéroaromatique à 5 chaînons contenant jusqu'à 4 hétéroatomes dont un ou plusieurs quelconques peuvent être N, et dont l'un peut être O ou S, et qui peut être éventuellement substitué par un groupe alcoyle en $C_{1-4}$ éventuellement substitué par un groupe $CO_2R^4$ ou $OR^5$.

2. Composé suivant la revendication 1 de formule (II):

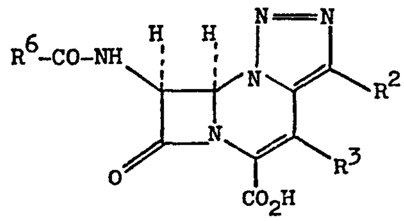

(II)

ou sel de ce composé, formule dans laquelle $R^2$ et $R^3$ ont la signification indiquée pour la formule (I) dans la revendication 1 et $R^6$ est un groupe tel que $R^6$—CO—NH— soit un groupe acylamino tel qu'on le trouve dans les pénicillines et les céphalosporines à activité antibactérienne.

3. Composé suivant la revendication 2, caractérisé en ce que $R^3$ est un groupe méthyle.

4. Composé suivant la revendication 2 de formule (III):

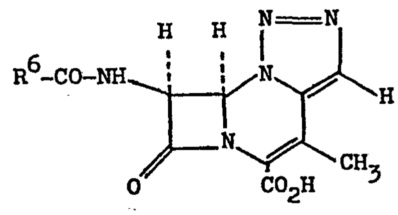

(III)

ou sel de ce composé, formule dans laquelle $R^6$ a la même signification que dans la formule (II) dans la revendication 2.

5. Composé suivant la revendication 2, caractérisé en ce que $R^6$.CO répond à l'une des sous-formules (a)—(d):

$$A_1 - (CH_2)_n - \underset{\displaystyle X}{\underset{|}{CH}} - (CH_2)_m - CO \qquad (a)$$

$$A_2 - CO \qquad (b)$$

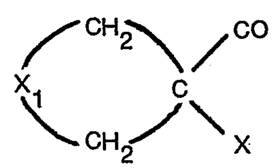

(c)

$$A_2 - X_2 - (CH_2)_n - CO \qquad (d)$$

dans lesquelles n est 0, 1 ou 2; m est 0, 1 ou 2; $A_1$ est un groupe alcoyle en $C_1$—$C_6$, cycloalcoyle en $C_3$—$C_6$, cyclohexényle, cyclohexadiényle, phényle, hydroxyphényle, thiényle ou pyridyle; X est un atome d'hydrogène ou d'halogène, un groupe acide carboxylique, ester carboxylique, azido, tétrazolyle, hydroxy, acyloxy, amino, uréido, guanidino ou acyluréido; $A_2$ est un groupe phényle, 2-diméthoxyphényle, 2-alcoxy-1-naphtyle, 3-arylisoxazolyle ou 3-aryl-5-méthylisoxazolyle; $X_1$ est un groupe $CH_2OCH_2$, $CH_2SCH_2$ ou $(CH_2)_n$; et $X_2$ est un atome d'oxygène ou de soufre.

6. Composé suivant la revendication 5, caractérisé en ce que $R^6$ est un groupe de sous-formule (e) ou (f):

$$R^7—\underset{\displaystyle R^8}{\underset{|}{CH}}— \qquad (e)$$

$$R^9—\underset{\displaystyle R^{10}}{\underset{|}{CH}}— \qquad (f)$$

dans lesquelles $R^7$ est un groupe phényle, thiényle ou phénoxy; $R^8$ est un atome d'hydrogène ou un groupe méthyle; $R^9$ est un groupe phényle, p-hydroxyphényle, thiényle ou cyclohexadiényle; et $R^{10}$ est un groupe hydroxy, amino ou acide carboxylique ou un ester d'alcoyle inférieur ou de phényle, de tolyle ou d'indanyle de celui-ci.

7. Composé suivant la revendication 6, caractérisé en ce que $R^6CO$ est un groupe D-phénylglycyle, D-p-hydroxyphénylglycyle, D-mandélyle, malonyle, benzoyle, 2-thiénylacétyle, 3-thiénylacétyle, 2-thiényl-$\alpha$-carboxyacétyle, 3-thiényl-carboxyacétyle ou phénoxyacétyle.

8. Composé suivant la revendication 1, caractérisé en ce que $R^1$ est un atome d'hydrogène.

9. Composition pharmaceutique, caractérisée en ce qu'elle contient un composé suivant la revendication 2 et un véhicule ou excipient pharmaceutiquement acceptable pour celui-ci.

10. Procédé pour la préparation d'un composé suivant la revendication 2, caractérisé en ce qu'on effectue l'acylation d'un composé de formule (V):

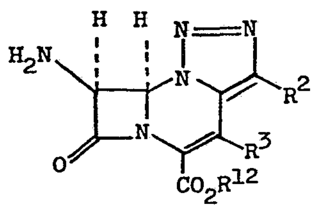

(V)

dans laquelle $R^2$ et $R^3$ sont tels que définis dans la revendication 2 et $R^{12}$ est un ester aisément convertible en un acide carboxylique ou en un sel de celui-ci par hydrogénolyse ou hydrolyse; avec un dérivé d'acylation réactif d'un acide carboxylique de formule (VI):

$$R^6—CO_2H \qquad (VI)$$

dans laquelle $R^6$ a la même signification que dans le cas de la formule (II) dans la revendication 2 et dans lequel tout groupe pouvant être acylé est éventuellement protégé, puis on effectue une ou plusieurs des réactions suivantes:

(a) élimination de tout groupe de protection à partir du groupe $R^6$,

(b) hydrolyse ou hydrogénolyse du groupe ester $CO_2R^{12}$ pour donner un groupe carboxylique libre ou salifié.

11. Procédé pour la preparation d'un composé de formule (V) suivant la revendication 10, caractérisé en ce qu'on effectue la détritylation d'un composé correspondant de formule (IV):

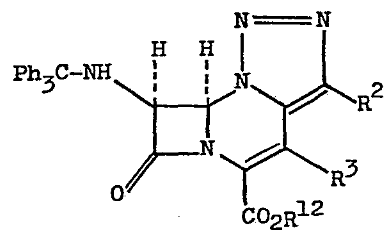

(IV)

dans laquelle $R^2$, $R^3$ et $R^{12}$ ont la même signification que dans la revendication 10.

**Patentansprüche**

1. Eine Verbindung der Formule (I):

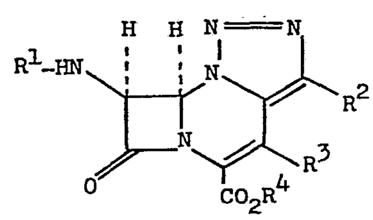

(I)

in der $R_1$ eine Wasserstoffatom, eine Tritylgruppe oder ein Acylrest ist, wie er in antibakteriell wirksamen Penicillinen oder Cephalosporinen zu finden ist, $R_2$ ein Wasserstoffatom, ein Alkylrest mit 1 bis 4 Kohlenstoffatomen, die Phenyl- oder Pyridylgruppe oder ein Phenylrest ist, der durch ein Fluor-, Chlor- oder Bromatom, die Nitrogruppe, einen Alkylrest mit 1 bis 4 Kohlenstoffatomen, einen Alkoxyrest mit 1 bis 4 Kohlenstoffatomen, die Carboxylgruppe oder deren $C_{1-4}$-Alkyl- oder Benzylester substituiert ist, $R_4$ eine solche Gruppe ist, daß $CO_2R^4$ die Carboxylgruppe oder deren Salz oder leicht entfernbarer Ester ist,

$R_3$ ein Wasserstoffatom oder ein $C_{1-4}$-Alkylrest ist, der gegebenenfalls durch eine Gruppe $CO_2R^4$, $OR^5$ oder $SR^7$ substituiert ist, wobei $R_4$, wie vorstehend beschrieben, definiert ist, $R^5$ ein $C_{1-4}$-Alkylrest oder ein $C_{1-4}$-Acylrest ist und $R_7$ ein 5-gliedriger heteroaromatischer Ring ist, der bis zu 4 Heteroatome enthält, von denen jedes Stickstoff sein kann und von denen eines Sauerstoff oder Schwefel sein kann, und der gegebenenfalls durch eine $C_{1-4}$-Alkylgruppe substituiert sein kann, die gegebenenfalls durch eine Gruppe $CO_2R^4$ oder $OR^5$ substituiert ist.

2. Eine Verbindung nach Anspruch 1 der allgemeinen Formel (II):

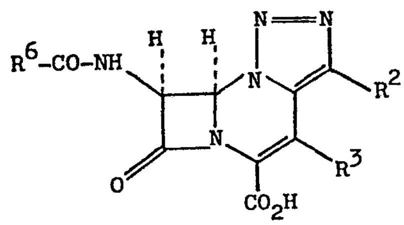
(II)

oder deren Salz, in der $R^2$ und $R^3$ wie in Bezug auf die Formel (I) in Anspruch 1 definiert sind und $R^6$ eine solche Gruppe ist, daß $R^6$—CO—NH— ein Acylaminorest ist, wie er in antibakteriell wirksamen Penicillinen oder Cephalosporinen zu finden ist.

3. Eine Verbindung nach Anspruch 2, in der $R^3$ die Methylgruppe ist.

4. Eine Verbindung nach Anspruch 2 der Formel (III):

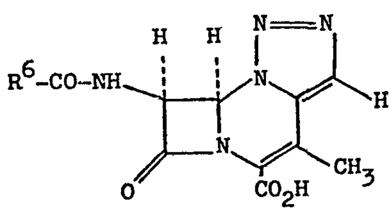
(III)

oder deren Salz, in der $R^6$ wie in Bezug auf die Formel (II) in Anspruch 2 definiert ist.

5. Eine Verbindung nach Anspruch 2, in der $R^6$.CO eine der Unterformeln (a) — (d) ist:

$$A_1 - (CH_2)_n - \underset{\displaystyle X}{\underset{|}{CH}} - (CH_2)_m - CO \qquad (a)$$

$$A_2 - CO \qquad (b)$$

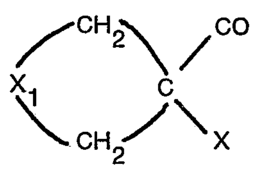
(c)

$$A_2 - X_2 - (CH_2)_n - CO \qquad (d)$$

in denen n 0, 1 oder 2 ist, m 0, 1 oder 2 ist, $A_1$ ein $C_1$—$C_6$-Alkyl-, $C_3$—$C_6$-Cycloalkyl-, Cyclohexenyl-, Cyclohexadienyl-, Phenyl-, Hydroxyphenyl-, Thienyl- oder Pyridylrest ist, X ein Wasserstoff- oder Halogenatom, die Carboxyl-, Carboxylester-, Azido-, Tetrazolyl-, Hydroxy-, Acyloxy-, Amino-, Ureido-, Guanidino- oder Acylureidogruppe ist, $A_2$ die Phenyl-, 2,6-Dimethoxyphenyl-, 2-Alkoxy-1-naphthyl-, 3-Arylisoxazoly- oder 3-Aryl-5-methylisoxazolylgruppe ist, $X_1$ die Gruppe $CH_2OCH_2$, $CH_2SCH_2$ oder $(CH_2)_n$ ist und $X_2$ ein Sauerstoff- oder Schwefelatom ist.

6. Eine Verbindung nach Anspruch 5, in der $R^6$ ein Rest der Unterformeln (e) oder (f) ist:

$$R^7\text{—}\underset{\displaystyle R^8}{\underset{|}{CH}}\text{—} \qquad (e)$$

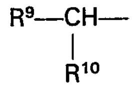

(f)

in denen $R^7$ die Phenyl-, Thienyl- oder Phenoxygruppe ist, $R^8$ ein Wasserstoffatom oder die Methylgruppe ist, $R^9$ die Phenyl-, p-Hydroxyphenyl-, Thienyl- oder Cyclohexadienylgruppe ist und $R^{10}$ die Hydroxyl-, Amino- oder Carboxylgruppe oder deren (nieder)-Alkyl-, Phenyl-, Tolyl- oder Indanylester ist.

7. Eine Verbindung nach Anspruch 6, in der $R^6CO$ die D-Phenylglycyl-, D-p-Hydroxyphenylglycyl-, D-Mandelyl-, Malonyl-, Benzoyl-, 2-Thienylacetyl-, 3-Thienylacetyl-, 2-Thienyl-$\alpha$-carboxyacetyl-, 3-Thienyl-$\alpha$-carboxacetyl- oder Phenoxyacetylgruppe ist.

8. Eine Verbindung nach Anspruch 1, in der $R^1$ ein Wasserstoffatom ist.,

9. Ein pharmazeutisches Präparat, das eine Verbindung wie in Anspruch 2 und ein pharmakologisch verträgliches Trägermaterial hierfür enthält.

10. Verfahren zur Herstellung einer Verbindung, wie in Anspruch 2 beansprucht, wobei das Verfahren die Maßnahme einer Acylierung einer Verbindung der Formel (V):

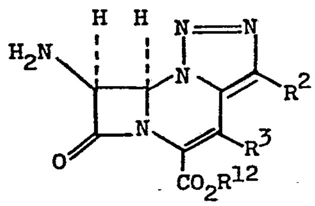

(V)

in der $R^2$ und $R^3$ wie in Anspruch 2 definiert sind und $R^{12}$ ein leicht in eine Carbonsäure oder deren Salz durch Hydrogenolyse oder Hydrolyse umwandelbarer Ester ist, mit einem reaktionsfähigen Acylierungsderivat einer Carbonsäure der Formel (VI):

$$R^6—CO_2H \qquad (VI)$$

in der $R^6$ wie in Bezug auf die Formel (II) in Anspruch 2 Definiert ist und in der gegebenenfalls eine Acylat-bildende Gruppe geschützt ist, und danach die Durchführung einer oder mehrerer der nachstehenden Umsetzungen Umsetzungen umfaßt:

(a) Entfernen der Schutzgruppe am Rest $R^6$,

(b) Hydrolysieren oder Hydrogenolysieren der Estergruppe $CO_2R^{12}$ zur Bildung einer freien Carbonsäure oder deren Salz.

11. Verfahren zur Herstellung einer Verbindung der Formel (V) in Anspruch 10, die die Maßnahme einer Detritylierung einer entsprechenden Verbindung der Formel (IV):

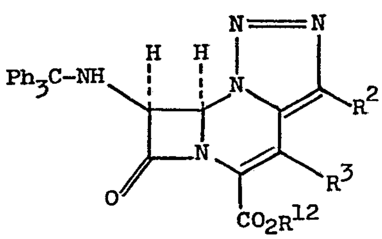

(IV)

umfaßt, in der $R^2$, $R^3$ und $R^{12}$ wie in Anspruch 10 definiert sind.